(19) Europäisches Patentamt
European Patent Office
Office européen des brevets

(11) EP 2 238 112 B1

(12) # FASCICULE DE BREVET EUROPEEN

(45) Date de publication et mention de la délivrance du brevet:
**31.07.2013 Bulletin 2013/31**

(21) Numéro de dépôt: **09706632.8**

(22) Date de dépôt: **27.01.2009**

(51) Int Cl.:
***C07D 221/14*** *(2006.01)* ***G01T 1/204*** *(2006.01)*

(86) Numéro de dépôt international:
**PCT/EP2009/050865**

(87) Numéro de publication internationale:
**WO 2009/095376 (06.08.2009 Gazette 2009/32)**

(54) **DERIVES DU 1,8-NAPHTALIMIDE EN TANT QU'AGENTS DE SCINTILLATION, NOTAMMENT POUR LA DISCRIMINATION ENTRE LES NEUTRONS RAPIDES ET LES RAYONS GAMMA**

DERIVATE VON 1,8-NAPHTHALIMID ALS SZINTILLATIONSMITTEL, INSBESONDERE ZUR UNTERSCHEIDUNG ZWISCHEN SCHNELLEN NEUTRONEN UND GAMMASTRAHLEN

DERIVATIVES OF 1,8-NAPHTHALIMIDE AS SCINTILLATION AGENTS, ESPECIALLY FOR THE DISCRIMINATION BETWEEN FAST NEUTRONS AND GAMMA RAYS

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **31.01.2008 FR 0850611**

(43) Date de publication de la demande:
**13.10.2010 Bulletin 2010/41**

(73) Titulaire: **Commissariat à l'Énergie Atomique et aux Énergies Alternatives**
**75015 Paris (FR)**

(72) Inventeurs:
- **HAMEL, Matthieu**
  **F-50100 Cherbourg (FR)**
- **NORMAND, Stéphane**
  **F-78200 Mantes La Jolie (FR)**
- **SIMIC, Vesna**
  **F-92370 Chaville (FR)**

(74) Mandataire: **Ilgart, Jean-Christophe**
**BREVALEX**
**95 rue d'Amsterdam**
**75378 Paris Cedex 8 (FR)**

(56) Documents cités:
- **VASIL'CHENKO V. G. ; GOLOVKIN S. V. ; ZIMIN K. V. ; ET ALL: "NEW LIQUID SCINTILLATORS FOR DETECTORS BASED ON CAPILLARY FIBERS" INSTRUMENTS AND EXPERIMENTAL TECHNIQUES, vol. 40, no. 2, 1997, pages 175-185, XP008097769**
- **BERESNEV, V. I., ET ALL: "Spectrum-shifting lightguides for large scintillation and Cerenkov detectors" JOURNAL ATOMIC ENERGY, vol. 64, no. 3, 1988, pages 268-274, XP008097768**
- **VASIL'CHENKO, V. G.; LAPSHIN, V. G.; PERESYPKIN, A. I.; KONSTANTINCHENKO,A. A.; ET ALL.: "New results on radiation damage studies of plastic scintillators" NUCLEAR INSTRUMENTS & METHODS IN PHYSICS RESEARCH, SECTION A: ACCELERATORS, SPECTROMETERS, DETECTORS, AND ASSOCIATED EQUIPMENT, vol. 369, 1996, pages 55-61, XP004003164**
- **BRITVICH, G. I.; PERESYPKIN, A. I.; RYKALIN, V. I.; VASIL'CHENKO, V. G.; ET ALL.: "INVESTIGATION OF RADIATION RESISTANCE OF POLYSTYRENE-BASED SCINTILLATORS" INSTRUMENTS AND EXPERIMENTAL TECHNIQUES, vol. 36, no. 1, 1993, pages 74-80, XP008097800**
- **BRITVICH, G. I.; PERESYPKIN, A. I.; RYKALIN, V. I.; VASIL'CHENKO, V. G.; ET ALL.: "Radiation damage studies on polystyrene-based scintillators" NUCLEAR INSTRUMENTS & METHODS IN PHYSICS RESEARCH, SECTION A: ACCELERATORS, SPECTROMETERS, DETECTORS, AND ASSOCIATED EQUIPMENT, vol. 326, 1993, pages 483-488, XP002501673**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 238 112 B1

## Description

### DOMAINE TECHNIQUE

L'invention se rapporte à l'utilisation de dérivés du 1,8-naphtalimide comme agents de scintillation et, plus spécialement, comme agents de discrimination entre les neutrons rapides et les rayons gamma.

Elle se rapporte également à des scintillateurs liquides comprenant ces agents de scintillation en solution dans un solvant ainsi qu'à de nouveaux dérivés du 1,8-naphtalimide utiles comme agents de scintillation, notamment pour la discrimination entre les neutrons rapides et les rayons gamma.

L'invention est susceptible de trouver application dans tous les domaines où sont utilisés des scintillateurs et, en particulier :

* dans le domaine industriel, par exemple pour la mesure de paramètres physiques de pièces en cours de fabrication, pour l'inspection non destructrice de matériaux, pour le contrôle de la radioactivité aux points d'entrée et de sortie de sites sensibles et pour le contrôle de déchets radioactifs ;
* dans le domaine géophysique, par exemple pour l'évaluation de la radioactivité naturelle des sols ;
* dans le domaine de la physique fondamentale et, en particulier, de la physique nucléaire ;
* dans le domaine de la sécurité des biens et des personnes, par exemple pour la sécurité d'infrastructures critiques, le contrôle de marchandises en circulation (bagages, conteneurs, véhicules, ...) ainsi que pour la radioprotection des travailleurs des secteurs industriel, nucléaire et médical ; et
* dans le domaine de l'imagerie médicale qui représente aujourd'hui l'un des domaines majeurs d'application des scintillateurs.

### ÉTAT DE LA TECHNIQUE ANTÉRIEURE

La discrimination entre les neutrons rapides et les rayons gamma, plus simplement dénommée « discrimination n/$\gamma$ », est un procédé qui permet de différencier les contributions respectives des interactions entre les neutrons rapides et les rayons gamma avec un scintillateur organique.

Cette discrimination est rendue possible par la différence de forme du signal rendu par le scintillateur lors de l'interaction rayonnement/ matière.

Il existe essentiellement deux types de scintillateurs organiques capables de discriminer les neutrons rapides des rayons gamma : des scintillateurs organiques solides et des scintillateurs organiques liquides.

Les scintillateurs organiques solides, qui sont fabriqués à partir de monocristaux, par exemple de stilbène ou d'anthracène, permettent généralement une bonne discrimination n/$\gamma$ mais la réponse dépend de l'angle d'incidence des particules. Il est, par ailleurs, difficile de préparer des scintillateurs organiques solides de taille importante en raison du coût de fabrication élevé de tels scintillateurs.

Les scintillateurs organiques liquides, qui présentent, eux, l'avantage d'une grande disponibilité et d'un faible coût de fabrication même en volumes importants, sont constitués d'un mélange complexe de plusieurs composés.

Ce mélange comprend généralement un ou plusieurs solvants organiques et au moins deux fluorophores : un fluorophore primaire qui a pour rôle de transformer une énergie électronique en lumière détectable et un fluorophore secondaire dit « wavelength shifter » qui a pour rôle d'augmenter la longueur d'onde d'émission du scintillateur pour augmenter son rendement de détection par les photodétecteurs. D'autres composés peuvent éventuellement être ajoutés tels que des surfactants, des extractants ou des charges pour des usages particuliers du scintillateur.

Actuellement, les scintillateurs organiques liquides les plus utilisés sont le NE213, le BC-501A de Bicron et l'Ultima Gold® AB de Perkin Elmer.

De par la complexité de leur composition, ces scintillateurs liquides ont de mauvaises propriétés de vieillissement et doivent donc être conditionnés et stockés dans des conditions très strictes. Ils sont, par ailleurs, extrêmement sensibles aux gaz et, en particulier, à l'oxygène de l'air susceptible de s'y dissoudre, d'où la nécessité de les soumettre fréquemment à des bullages de gaz inertes (argon) pour éviter les phénomènes de quenching (diminution du rendement de fluorescence).

Les Inventeurs se sont donc fixé pour but de fournir de nouveaux agents de scintillation qui, outre d'être capables de discriminer les neutrons des rayons gamma, permettent de réaliser des scintillateurs organiques solides ou liquides qui, de manière générale, soient dénués des inconvénients présentés par les scintillateurs organiques solides et liquides utilisés à ce jour pour réaliser une telle discrimination.

En particulier, les Inventeurs se sont fixé pour but que ces agents de scintillation permettent de réaliser des scintillateurs liquides qui, tout en ayant des propriétés spectroscopiques au moins aussi intéressantes que celles des scintillateurs liquides actuellement disponibles, soient suffisamment stables pour ne nécessiter aucune précaution particulière pour leur conditionnement et leur stockage, et soient insensibles à la présence de gaz dissous et, notamment,

d'oxygène dissous.

Ils se sont encore fixé pour but que la synthèse de ces agents de scintillation et la préparation de scintillateurs, solides ou liquides, à partir de ceux-ci soient faciles à mettre en oeuvre et ne fassent appel qu'à des manipulations classiquement utilisées en chimie organique.

## EXPOSÉ DE L'INVENTION

Ces buts et d'autres encore sont atteints par l'invention qui propose, en premier lieu, l'utilisation d'un dérivé du 1,8-naphtalimide répondant à la formule générale (I) ci-après :

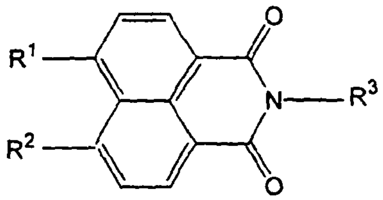

(I)

dans laquelle :

- $R^1$ représente un groupe choisi parmi :
  - les groupes -OR' et -SR' où R' représente un groupe hydrocarboné linéaire ou ramifié, saturé ou insaturé, en $C_1$ à $C_{20}$, éventuellement substitué, ou un groupe hydrocarboné cyclique saturé en $C_3$ à $C_{10}$, éventuellement substitué, ou encore un groupe aryle ou hétéroaryle éventuellement substitué ; et
  - les groupes -NR'R" où R' a la même signification que précédemment, tandis que R" représente soit un atome d'hydrogène, soit un groupe hydrocarboné linéaire ou ramifié, saturé ou insaturé, en $C_1$ à $C_{20}$, éventuellement substitué, soit un groupe hydrocarboné cyclique saturé en $C_3$ à $C_{10}$, éventuellement substitué, soit encore un groupe aryle ou hétéroaryle éventuellement substitué;
- $R^2$ représente un atome d'hydrogène ou un groupe identique ou différent de $R^1$, choisi parmi :
  - les groupes -OR' et -SR' où R' représente un groupe hydrocarboné linéaire ou ramifié, saturé ou insaturé, en $C_1$ à $C_{20}$, éventuellement substitué, ou un groupe hydrocarboné cyclique saturé en $C_3$ à $C_{10}$, éventuellement substitué, ou encore un groupe aryle ou hétéroaryle éventuellement substitué ; et
  - les groupes -NR'R" où R' a la même signification que précédemment, tandis que R" représente soit un atome d'hydrogène, soit un groupe hydrocarboné linéaire ou ramifié, saturé ou insaturé, en $C_1$ à $C_{20}$, éventuellement substitué, soit un groupe hydrocarboné cyclique saturé en $C_3$ à $C_{10}$, éventuellement substitué, soit encore un groupe aryle ou hétéroaryle éventuellement substitué;

  tandis que
- $R^3$ représente :
  - un groupe acyle, ou
  - un groupe hydrocarboné linéaire ou ramifié, saturé ou insaturé, en $C_1$ à $C_{20}$, éventuellement substitué, ou
  - un groupe hydrocarboné cyclique saturé en $C_3$ à $C_{10}$, éventuellement substitué, ou
  - un groupe hétérocyclique saturé en $C_3$ à $C_{10}$, éventuellement substitué, ou encore
  - un groupe aryle ou hétéroaryle éventuellement substitué ;

ou de l'un de ses sels, en tant qu'agent de scintillation pour discriminer les neutrons rapides des rayons gamma.

Les Inventeurs ont, en effet, constaté qu'en substituant le 1,8-naphtalimide en position 4 et/ou en position 5 avec un groupe $R^1$ et/ou $R^2$ tel(s) que précédemment défini(s), on obtient des composés fluorescents qui présentent des propriétés spectroscopiques telles qu'il est possible de les utiliser comme agents scintillants et, notamment, comme agents de discrimination n/γ, et de réaliser avec ces composés des scintillateurs organiques liquides à un seul fluorophore, qui sont à la fois stables dans le temps et insensibles aux gaz dissous.

Dans ce qui précède et ce qui suit, on entend par « groupe hydrocarboné linéaire ou ramifié, saturé ou insaturé, en $C_1$ à $C_{20}$ », tout groupe alkyle, alcényle ou alcynyle qui comprend au moins 1 atome de carbone mais pas plus de 20 atomes de carbone. Un tel groupe est, par exemple, un groupe méthyle, éthyle, propyle, isopropyle, butyle, pentyle,

néopentyle, hexyle, éthylényle, propylényle, buténylе, penténylе, hexénylе, méthylpenténylе, buta-1,3-diénylе, éthynylе, propynylе, butynylе, pentynylе, hexynylе, etc.

On entend par « groupe hydrocarboné cyclique saturé en $C_3$ à $C_{10}$ », tout groupe qui est formé d'un cycloalkyle ou de plusieurs cycloalkyles fusionnés et qui comprend au moins 3 atomes de carbone mais pas plus de 10 atomes de carbone. Un tel groupe est, par exemple, un groupe cyclopropyle, cyclobutyle, cyclopentyle, cyclohexyle, bicyclohexyle, bicyclodécyle, etc.

On entend par « groupe hétérocyclique saturé en $C_3$ à $C_{10}$ », un groupe monocyclique ou polycyclique qui comporte un ou plusieurs hétéroatomes et qui comprend au moins 3 atomes de carbone mais pas plus de 10 atomes de carbone. Un tel groupe est, par exemple, un groupe tétrahydrofuryle, tétrahydrothiophényle, pyrrolidinyle, pipéridyle, dioxanyle, etc.

On entend par « groupe aryle », un groupe monocyclique ou polycyclique qui satisfait à la règle de Hückel, c'est-à-dire qui présente un nombre d'électrons $\pi$ délocalisés égal à $4n + 2$ (avec $n = 0, 1, 2, 3, ...$), et par « groupe hétéroaryle », un groupe tel qu'il vient d'être défini mais qui comprend un ou plusieurs hétéroatomes. A titre d'exemples de groupe aryle susceptible d'être utilisé, on peut citer les groupes cyclopentadiényle, phényle, benzyle, biphényle, pyrényle, naphtalényle, phénantrényle et anthracényle, tandis qu'à titre d'exemples de groupe hétéroaryle, on peut citer les groupes furanyle, pyrrolyle, thiophényle, oxazolyle, pyrazolyle, thiazolyle, imidazolyle, triazolyle, pyridinyle, pyranyle, quinoléinyle, pyrazinyle et pyrimidinyle.

Enfin, on entend par « hétéroatome », tout atome autre que de carbone ou d'hydrogène comme, par exemple, un atome d'oxygène, de soufre, d'azote, de phosphore ou encore de bore, étant entendu, toutefois, que les hétéroatomes susceptibles d'être impliqués dans des cycles sont des atomes d'oxygène, d'azote ou de soufre.

Conformément à l'invention, on préfère utiliser comme groupes hydrocarbonés cycliques saturés en $C_3$ à $C_{10}$ et comme groupes hétérocycliques saturés en $C_3$ à $C_{10}$, des groupes monocycliques à 5 ou 6 chaînons.

De manière similaire, on préfère utiliser comme groupes aryles ou hétéroaryles, des groupes monocycliques à 5 ou 6 chaînons ou des groupes polycycliques ne comportant pas plus de 3 cycles et, mieux encore, pas plus de 2 cycles, à 5 ou 6 chaînons chacun.

Selon une première disposition préférée de l'invention, le dérivé du 1,8-naphtalimide présente une longueur d'onde maximale d'émission comprise entre 350 et 550 nm et, mieux encore, entre 380 et 470 nm.

C'est la raison pour laquelle la préférence revient aux dérivés du 1,8-naphtalimide répondant à la formule générale (I) dans laquelle $R^1$ représente un groupe -OR' ou -SR' où R' a la même signification que précédemment, tandis que $R^2$ représente un atome d'hydrogène, dont la longueur d'onde maximale d'émission se situe autour de 420 nm.

De manière particulièrement préférée, $R^1$ représente un groupe alcoxy, alcényloxy ou alkylsulfanyle en $C_1$ à $C_{20}$ et, mieux encore, en $C_1$ à $C_{10}$ comme, par exemple, un groupe méthoxy, éthoxy, propoxy, butyloxy, pentoxy, octyloxy, vinyloxy, allyloxy, buténylоxy, hexényloxy, méthylsulfanyle, éthylsulfanyle, propylsulfanyle, butylsulfanyle, hexylsulfanyle ou octylsulfanyle.

Selon une autre disposition préférée de l'invention, $R^3$ représente un groupe relativement volumineux et encombrant d'un point de vue stérique de manière à optimiser le rapport signal-bruit. Aussi préfère-t-on que $R^3$ représente un groupe cyclique, typiquement un groupe aryle ou hétéroaryle, substitué en positions 2 et 5 par rapport à l'atome d'azote du noyau naphtalimide par un groupe alkyle ramifié en $C_3$ à $C_6$ comme, par exemple, un groupe isopropyle ou t-butyle. Un groupe cyclique de ce type est, par exemple, le groupe di-t-butylphényle.

Toutefois, des dérivés du 1,8-naphtalimide ayant pour radical $R^3$, une simple chaîne alkyle linéaire comme, par exemple, une chaîne n-butyle, se sont également révélés intéressants.

Selon encore une autre disposition préférée de l'invention, le dérivé du 1,8-naphtalimide est choisi parmi les dérivés 4, 5, 6, 7, 8, 9 et 12 représentés ci-après :

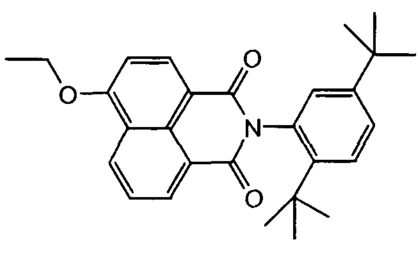

**4**

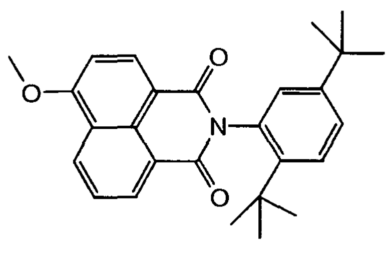

**5**

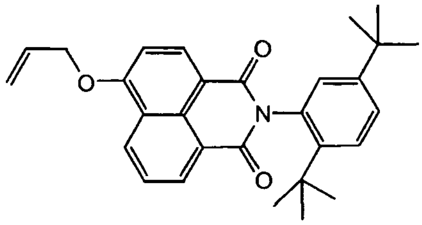

**6**

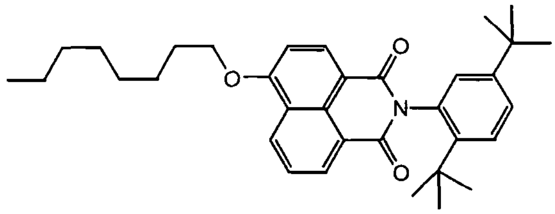

**7**

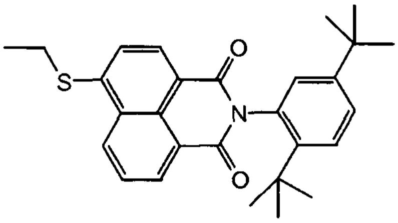

**8**

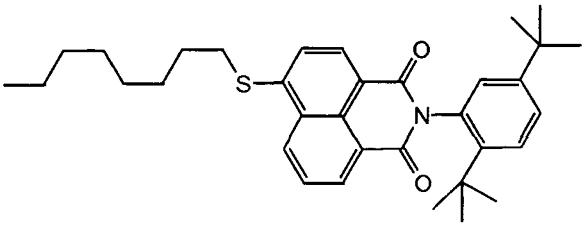

**9**

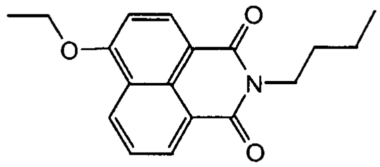

**12**

Parmi ces dérivés, on préfère tout particulièrement le dérivé **4.**

Conformément à l'invention, le dérivé du 1,8-naphtalimide est susceptible d'être utilisé comme agent de scintillation aussi bien sous une forme solide, c'est-à-dire en étant incorporé dans une matrice solide, que sous une forme liquide, c'est-à-dire en solution dans un solvant.

Des matrices solides dans lesquelles ce dérivé peut être incorporé sont notamment :

- des matrices polymères, obtenues par polymérisation en chaîne ou polycondensation, auquel cas le polymère ou le copolymère est avantageusement choisi parmi les (co)polymères vinyliques (polystyrènes, poly(vinyltoluènes), poly(vinylxylènes), ...), les (co)poly(méth)acrylates, les (co)poly(méth)-acrylamides, les (co)poly(méth)acrylonitriles et les (co)polysiloxanes ; le dérivé du 1,8-naphtalimide peut être incorporé dans la matrice soit par simple dispersion, soit être greffé aux chaînes par (co)polymérisation ;
- des matrices obtenues par la technologie sol-gel du type de celles décrites par B. Kesanli et al. dans Appl. Phys. Lett. 2006, 89, 214104 ;
- des matrices constituées d'un ou plusieurs hydrocarbures (saturés ou insaturés) solides à température ambiante comme, par exemple, des matrices d'éicosane ou de triacontane ; et
- des matrices constituées d'un mélange de cristaux liquides comme, par exemple, celles décrites par M. I. Barnik et al. dans Nucl. Instrum. Methods Phys. Res., Sect. A 2000, 449, 537-545.

Des solvants dans lesquels le dérivé du 1,8-naphtalimide peut être mis en solution sont principalement les solvants organiques aromatiques comme, par exemple, le xylène, le benzène, le toluène, le mésitylène, le pseudocumène ou le *p*-isopropylbiphényle, et leurs mélanges, le toluène étant particulièrement préféré. D'autres solvants dits de sécurité, possédant un point éclair élevé (d'environ 150°C), peuvent également être envisagés comme, par exemple, le dodécylbenzène (LAB), le (1-phényléthyl)-xylène (PXE) ou encore le bis(1-méthyléthyl)naphthalène (DIN).

Toutefois, il est également possible d'utiliser des solvants organiques non aromatiques comme les alcools ou les cétones, ou même de l'eau mélangée ou non à un solvant organique. Dans ce dernier cas toutefois, il convient que le dérivé se présente sous la forme d'un sel, par exemple un sel d'ammonium grâce à la présence d'un groupe amine tertiaire protoné, porté par l'un quelconque des radicaux $R^1$ à $R^3$.

Dans tous les cas, la concentration du dérivé du 1,8-naphtalimide dans le solvant est avantageusement au moins égale à 3 g/L. Cette concentration, qui peut aller jusqu'à la saturation du solvant en dérivé, est de préférence

comprise entre 8 et 12 g/L et est idéalement de 10 g/L.

Conformément à l'invention, le dérivé du 1,8-naphtalimide peut être utilisé conjointement avec un ou plusieurs agents dopants borés de manière à ce qu'il puisse aussi détecter les neutrons thermiques. De tels agents dopants sont, par exemple, l'o-carborane, les alkylborates comme le triméthylborate.

Egalement, le dérivé du 1,8-naphtalimide peut être utilisé conjointement avec un ou plusieurs agents propres à conférer à la matrice ou au solvant dans lequel il se trouve une meilleure tenue aux radiations. Un tel agent est, par exemple, l'oxyde de diphényle.

Quelle que soit la forme sous laquelle il est utilisé et quels que soient les éventuels agents auxquels il est associé, le dérivé du 1,8-naphtalimide présente de préférence un degré de pureté microanalytique, c'est-à-dire supérieur ou égal à 99,5%.

L'invention a aussi pour objet un scintillateur liquide qui comprend un dérivé du 1,8-naphtalimide répondant à la formule (I) représentée ci-après :

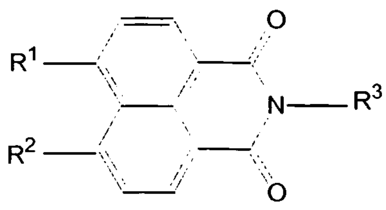

(I)

dans laquelle :

- $R^1$ représente un groupe choisi parmi :
  - les goupes - OR' et -SR' où R' représente un groupe hydrocarboné linéaire ou ramifié, saturé ou insaturé, en $C_1$ à $C_{20}$, éventuellement substitué, ou un groupe hydrocarboné cyclique saturé en $C_3$ à $C_{10}$, éventuellement substitué, ou encore un groupe aryle ou hétéroaryle éventuellement substitué ; et
  - les groupes -NR'R" où R' est tel que précédemment défini, tandis que R" représente soit un atome d'hydrogène, soit un groupe hydrocarboné linéaire ou ramifié, saturé ou insaturé, en $C_1$ à $C_{20}$, éventuellement substitué, soit un groupe hydrocarboné cyclique saturé en $C_3$ à $C_{10}$, éventuellement substitué, soit encore un groupe aryle ou hétéroaryle éventuellement substitué ;

- $R^2$ représente un atome d'hydrogène ou un groupe identique ou différent de $R^1$, choisi parmi :
  - les goupes - OR' et -SR' où R' représente un groupe hydrocarboné linéaire ou ramifié, saturé ou insaturé, en $C_1$ à $C_{20}$, éventuellement substitué, ou un groupe hydrocarboné cyclique saturé en $C_3$ à $C_{10}$, éventuellement substitué, ou encore un groupe aryle ou hétéroaryle éventuellement substitué ; et
  - les groupes -NR'R" où R' est tel que précédemment défini, tandis que R" représente soit un atome d'hydrogène, soit un groupe hydrocarboné linéaire ou ramifié, saturé ou insaturé, en $C_1$ à $C_{20}$, éventuellement substitué, soit un groupe hydrocarboné cyclique saturé en $C_3$ à $C_{10}$, éventuellement substitué, soit encore un groupe aryle ou hétéroaryle éventuellement substitué ;

- $R^3$ représente :
  - un groupe acyle, ou
  - un groupe hydrocarboné linéaire ou ramifié, saturé ou insaturé, en $C_1$ à $C_{20}$, éventuellement substitué, ou
  - un groupe hydrocarboné cyclique saturé en $C_3$ à $C_{10}$, éventuellement substitué, ou
  - un groupe hétérocyclique saturé en $C_3$ à $C_{10}$, éventuellement substitué, ou encore
  - un groupe aryle ou hétéroaryle éventuellement substitué ;

ou l'un de ses sels, en solution dans un solvant.

Dans ce scintillateur liquide, on préfère que le dérivé du 1,8-naphtalimide présente une longueur d'onde d'émission comprise entre 350 et 550 nm et, mieux encore, entre 380 et 470 nm.

Aussi, ce dérivé répond-t-il, de préférence, à la formule générale (I) dans laquelle $R^1$ représente un groupe -OR' ou -SR' où R' est tel que précédemment défini, tandis que $R^2$ représente un atome d'hydrogène.

Avantageusement, $R^1$ représente un groupe alcoxy, alcényloxy ou alkylsulfanyle en $C_1$ à $C_{20}$ et, mieux encore, en $C_1$ à $C_{10}$.

Par ailleurs, $R^3$ représente, de préférence, un groupe aryle ou hétéroaryle substitué en positions 2 et 5 par rapport à l'atome d'azote du noyau naphtalimide par un groupe alkyle ramifié en $C_3$ à $C_6$, avantageusement un groupe di-t-butylphényle.

Dans le scintillateur liquide, le dérivé du 1,8-naphtalimide est, de préférence, choisi parmi les dérivés **4, 5, 6, 7, 8, 9** et **12** précédemment représentés, le dérivé **4** étant, là encore, tout particulièrement préféré.

Par ailleurs, le solvant est, de préférence, un solvant organique aromatique du type xylène, benzène, toluène, mésitylène, pseudo-cumène, dodécylbenzène ou p-isopropylbiphényle, ou un mélange de tels solvants, le toluène étant particulièrement préféré.

Quant au dérivé du 1,8-naphtalimide, il est avantageusement présent à une concentration au moins égale à 3 g/L et, de préférence, comprise entre 8 et 12 g/L, une concentration idéale étant de 10 g/L.

Facultativement, le scintillateur liquide peut comprendre de plus un ou plusieurs agents dopants borés, par exemple du type de ceux précités, et/ou un ou plusieurs agents propres à lui conférer une meilleure tenue aux radiations, du type de ceux précédemment mentionnés.

Par contre, et c'est notamment l'un des grands avantages de l'invention, il n'est pas nécessaire de prévoir la présence d'un autre composé fluorescent dans le scintillateur liquide.

Parmi les dérivés du 1,8-naphtalimide susceptibles d'être utilisés comme agents de scintillation conformément à l'invention, certains sont connus en tant que composés chimiques, tandis que d'autres n'ont, à la connaissance des Inventeurs, jamais été décrits.

L'invention a donc encore pour objet un dérivé du 1,8-naphtalimide qui répond à la formule générale (I) représentée ci-avant et dans laquelle :

- $R^1$ représente un groupe choisi parmi les groupes -OR' et -SR' où R' représente un groupe hydrocarboné linéaire ou ramifié, saturé ou insaturé, en $C_1$ à $C_{20}$, éventuellement substitué, ou un groupe hydrocarboné cyclique saturé en $C_3$ à $C_{10}$, éventuellement substitué, ou encore un groupe aryle ou hétéroaryle éventuellement substitué ;
- $R^2$ représente un atome d'hydrogène ou un groupe qui est également choisi parmi les groupes -OR' et -SR' où R' est tel que précédemment défini ; tandis que
- $R^3$ représente un groupe aryle ou hétéroaryle substitué en positions 2 et 5 par rapport à l'atome d'azote du noyau naphtalimide par un groupe alkyle ramifié en $C_3$ à $C_6$ ;

et ses sels.

Dans ce dérivé, on préfère que :

* $R^2$ représente un atome d'hydrogène ;
* $R^1$ représente un groupe alcoxy, alcényloxy ou alkylsulfanyle en $C_1$ à $C_{20}$ et, mieux encore, en $C_1$ à $C_{10}$ ; et que
* $R^3$ représente un groupe di-t-butylphényle.

Conformément à l'invention, le dérivé du 1,8-naphtalimide est, de préférence, choisi parmi les dérivés **4, 5, 6, 7, 8** et **9** précédemment représentés, le dérivé **4** étant tout particulièrement préféré.

Les dérivés du 1,8-naphtalimide utiles comme agents de scintillation conformément à l'invention peuvent être préparés par des voies de synthèse à la portée de l'homme du métier.

En particulier, ces dérivés peuvent être synthétisés selon le schéma réactionnel général ci-après :

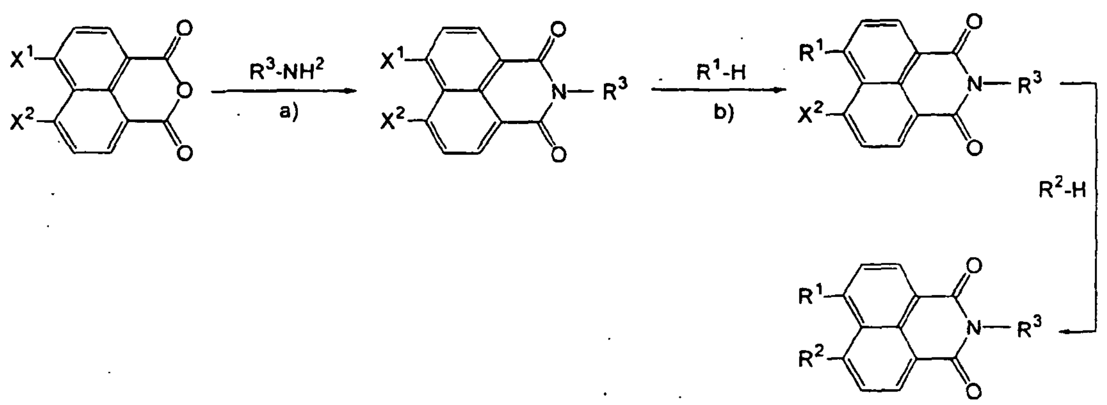


où :

- $x^1$ représente un atome d'halogène, typiquement de brome ;
- $x^2$ représente un atome d'hydrogène si l'on souhaite obtenir un dérivé du 1,8-naphtalimide de formule générale (I) dans laquelle $R^2$ représente un atome d'hydrogène, un atome d'halogène identique à $X^1$ si l'on souhaite obtenir un dérivé du 1,8-naphtalimide de formule générale (I) dans laquelle $R^2$ représente un groupe identique à $R^1$, et un groupe nitro si l'on souhaite obtenir un dérivé du 1,8-naphtalimide de formule générale (I) dans laquelle $R^2$ représente un groupe différent de $R^1$ ;
- la synthèse ne comprend que les étapes a) et b) si l'on souhaite obtenir un dérivé du 1,8-naphtalimide de formule générale (I) dans laquelle $R^2$ représente un atome d'hydrogène, tandis qu'elle comprend en plus l'étape c) si l'on souhaite obtenir un dérivé du 1,8-naphtalimide de formule générale (I) dans laquelle $R^2$ représente un groupe tel que précédemment défini les étapes b) et c) sont réalisées en même temps dans le cas où il est prévu que $R^1$ et $R^2$ soient le même groupe ;
- l'étape a) est une réaction classique d'amidation par une amine primaire, tandis que les étapes b) et c) sont des étapes de substitution nucléophile bien connues de l'homme du métier.

Si nécessaire, le dérivé du 1,8-naphtalimide ainsi obtenu est soumis à une ou plusieurs opérations de purification, par exemple par chromatographie sur gel de silice, pour obtenir un degré de pureté microanalytique.
D'autres caractéristiques et avantages de l'invention apparaîtront à la lecture du complément de description qui suit, qui se réfère à des exemples de synthèse de dérivés du 1,8-naphtalimide utiles comme agents de scintillation et de démonstration des propriétés de scintillateurs liquides préparés à partir de ces dérivés.
Bien entendu, ces exemples ne sont donnés qu'à titre d'illustrations de l'invention et n'en constituent en aucun cas une limitation.

## BRÈVE DESCRIPTION DES DESSINS

La figure 1 illustre les spectres d'absorption (courbe A) et d'émission (courbe B) d'un dérivé du 1,8-naphtalimide utile comme agent de scintillation.
La figure 2 illustre la discrimination n/γ telle qu'obtenue avec un scintillateur liquide selon l'invention, saturé en argon.
La figure 3 illustre la discrimination n/γ telle qu'obtenue avec un scintillateur liquide selon l'invention, saturé en oxygène.
La figure 4 illustre la discrimination n/γ telle qu'obtenue avec un premier scintillateur organique liquide du commerce, le NE213, saturé en argon.
La figure 5 illustre la discrimination n/γ telle qu'obtenue avec le NE213 saturé en oxygène.
La figure 6 illustre la discrimination n/γ telle qu'obtenue avec un autre scintillateur organique liquide du commerce, l'Ultima Gold® AB, saturé en argon.
La figure 7 illustre la discrimination n/γ telle qu'obtenue avec l'Ultima Gold® AB saturé en oxygène.
La figure 8 illustre la discrimination n/γ telle qu'obtenue avec encore un autre scintillateur organique liquide du commerce, le BC-501A, saturé en argon.
La figure 9 illustre la discrimination n/γ telle qu'obtenue avec le BC-501A saturé en oxygène.

## EXPOSÉ DÉTAILLÉ DE MODES DE RÉALISATION PARTICULIERS

### Exemple 1 : Synthèse de dérivés du 1,8-naphtalimide utiles comme agents de scintillation

#### 1.1. *N*-(2',5'-di-t-butylphényl)-4-éthoxy-1,8-naphtalimide

Le composé titre, qui correspond au dérivé **4** représenté ci-avant, est synthétisé en partant de l'anhydride 4-bromonaphtalique ou composé **1** et de la 2,5-di-t-butylaniline ou composé **2,** selon le schéma réactionnel suivant :

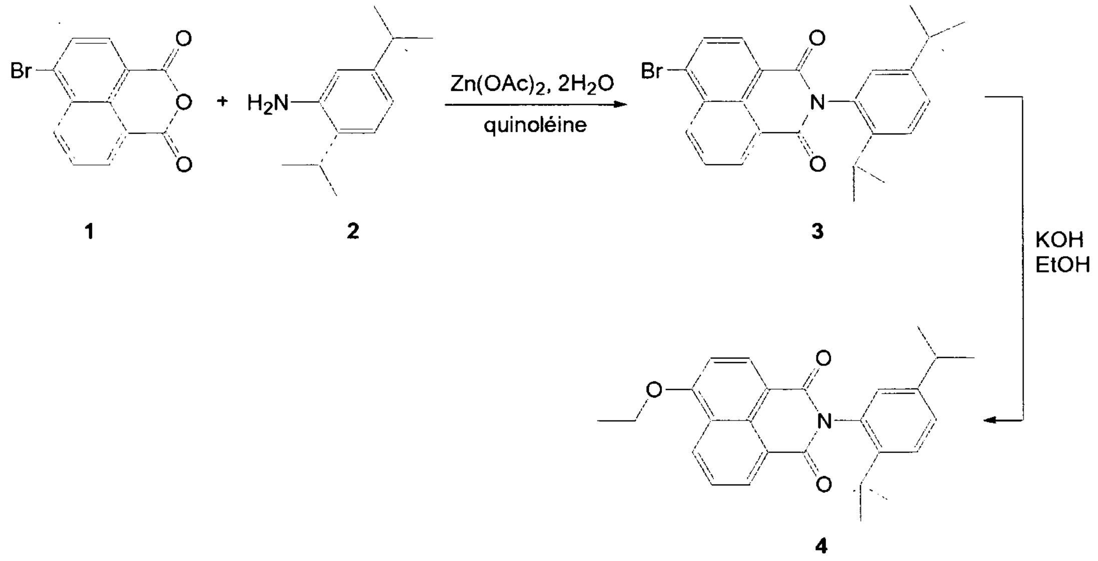


1 2 3 4

Les composés **1** et **2** sont disponibles commercialement.

<u>Synthèse du *N*-(2',5'-di-t-butylphényl)-4-bromo-1,8-naphtalimide ou composé **3**</u>

Dans un ballon de 250 mL équipé d'un réfrigérant à eau, 2,934 g (10,06 mmol) du composé **1** et 4,131 g (20,12 mmol) du composé **2** sont recouverts par 100 mL de quinoléine fraîchement distillée. Puis, 773 mg (3,52 mmol) d'acétate de zinc dihydraté sont ajoutés et le mélange réactionnel est chauffé au reflux du solvant pendant 5 heures. Après retour à température ambiante, le mélange est versé dans une solution aqueuse de pH = 1. La phase aqueuse est extraite au dichlorométhane. La phase organique est séchée, filtrée puis concentrée. Le résidu est finalement chromatographié sur gel de silice pour donner 4,583 g d'un solide beige (Rdt : 93%).

Point de fusion : 214°C (dec., heptane)

RMN $^1$H (250 MHz, $CDCl_3$) δ ppm : 1.19 (s, 9H, $CH_3$) ; 1.23 (s, 9H, $CH_3$) ; 6.91 (d, 1H, *J* = 2.2, $H_{6'}$) ; ; 7.37 (dd, 1H, *J* = 8.7, *J* = 2.2, $H_{4'}$) ; 7.48-7.52 (m, 1H, $H_{3'}$) ; 7.80 (dd, 1H, *J* = 8.5, *J* = 7.2, $H_6$) ; 7.99 (d, 1H, *J* = 8.0) ; 8.38 (d, 1H, *J* = 8.1) ; 8.55 (dd, 1H, *J* = 8.5, *J* = 1.3) ; 8.64 (dd, 1H, *J* = 7.2, *J* = 1.3)
RMN $^{13}$C (62.9 MHz) δ ppm : 31.2, 31.7, 34.2, 35.4, 122.6, 123.5, 126.3, 127.6, 128.1, 128.7, 129.4, 130.5, 130.8, 131.1, 131.6, 132.4, 132.5, 133.5, 143.7, 150.1, 164.52, 164.57
Infrarouge (neat, $cm^{-1}$) : 2960, 2873, 1666, 1589, 1496, 1357, 1234.

<u>Synthèse du dérivé **4**</u>

Dans un ballon de 25 mL, 1 g (2,15 mmol) du composé **3** et 0,154 g (2,36 mmol) d'hydroxyde de potassium sont solubilisés dans 10 mL d'éthanol. La solution est chauffée à 80°C au reflux pendant 5 heures. Après retour à température ambiante, le solvant est évaporé, le résidu directement purifié par chromatographie sur gel de silice puis le solide est recristallisé dans l'acétonitrile pour donner 775 mg du dérivé **4** sous la forme d'un solide blanc (Rdt : 84%).
Les spectres d'absorption et d'émission de ce dérivé sont illustrés sur la figure 1, la courbe A correspondant au spectre d'absorption et la courbe B correspondant au spectre d'émission.

Point de fusion : 196°C (acétonitrile)

RMN $^1$H (250 MHz, $CDCl_3$) δ ppm : 1.27 (s, 9H, $CH_3$) ; 1.31 (s, 9H, $CH_3$) ; 1.63 (t, 3H, $^3J$ = 6.9, $CH_3$) ; 4.38 (qd, 2H, $^3J$ = 7.0, $CH_2$-O) ; 6.99 (d, 1H, *J* = 2.1, $H_{6'}$) ; 7.06 (d, 1H, *J* = 8.3) ; 7.43 (dd, 1H, *J* = 8.5, *J* = 2.1) ; 7.57 (d, 1H, *J* = 8.5) ; 7.73 (t, 1H, *J* = 7.9) ; 8.58-8.67 (m, 3H)
RMN $^{13}$C (62.9 MHz, $CDCl_3$) δ ppm : 14.6, 30.3, 31.7, 34.2, 35.5, 64.8, 105.8, 115.2, 122.8, 123.8, 125.9, 126.0, 127.9, 128.6, 129.0, 129.9, 131.9, 133.2, 133.9, 143.8, 149.9, 160.4, 165.0, 165.6
Infrarouge (neat, $cm^{-1}$) : 2964, 2873, 1704, 1664, 1589, 1353, 1238
Analyse élémentaire : calculé (trouvé) pour $C_{28}H_{31}NO_3$, ½ $H_2O$ :

C : 76,68 % (77,1%)
H : 7,35% (7,3%)
N : 3,19% (3,7%)

**1.2. *N*-(2',5'-di-*t*-butylphényl)-4-méthoxy-1,8-naphtalimide**

Le composé titre, qui correspond au dérivé **5** représenté ci-avant, est synthétisé selon un protocole identique à celui décrit ci-dessus pour la synthèse du dérivé **4** à ceci près que la réaction entre le composé **3** et l'hydroxyde de potassium a lieu dans le méthanol au reflux, à 70°C pendant 5 heures. Après purification par chromatographie sur gel de silice puis recristallisation dans l'acétone, on obtient 166 du dérivé **5** sous la forme d'un solide blanc (Rdt : 62%) .

Point de fusion : 230°C (acétone)

RMN $^1H$ (250 MHz, $CDCl_3$) δ ppm : 1.27 (s, 9H, $CH_3$) ; 1.31 (s, 9H, $CH_3$) ; 4.15 (s, 3H, $CH_3$-O) ; 6.99 (d, 1H, *J* = 2.2, $H_{6'}$) ; 7.08 (d, 1H, *J* = 8.3) ; 7.43 (dd, 1H, *J* = 8.5, *J* = 2.2) ; 7.57 (d, 1H, *J* = 8.5) ; 7.74 (t, 1H, *J* = 7.6) ; 8.60-8.67 (m, 3H)
RMN $^{13}C$ (62.9 MHz, $CDCl_3$) δ ppm : 31.2, 31.7, 34.2, 35.5, 56.3, 105.2, 115.5, 122.8, 123.8, 126.0, 126.1, 127.8, 128.6, 128.9, 129.8, 132.0, 133.1, 133.9, 143.8, 149.9, 161.0, 164.9, 165.5
Infrarouge (neat, $cm^{-1}$) : 2960, 2869, 1700, 1666, 1591, 1355, 1236
Analyse élémentaire : calculé (trouvé) pour $C_{27}H_{29}NO_3$, ½ $H_2O$ :

C : 76,39% (76,7%)
H : 7,12% (7,4%)
N : 3,30% (3,6%).

**1.3. *N*-(2',5'-di-*t*-butylphényl)-4-allyloxy-1,8-naphtalimide**

Le composé titre, qui correspond au dérivé **6** représenté ci-avant, est synthétisé selon un protocole identique à celui décrit ci-dessus pour la synthèse du dérivé **4** à ceci près que la réaction entre le composé **3** et l'hydroxyde de potassium a lieu dans l'alcool allylique à 70°C pendant 16 heures. On obtient ainsi 344 mg du composé **6** sous la forme d'un solide jaune pâle (Rdt : 72%).

Point de fusion : 183-184°C

RMN $^1H$ (400 MHz) δ ppm : 1.27 (s, 9H, $CH_3$) ; 1.30 (s, 9H, $CH_3$) ; 4.87-4.89 (m, 2H, $CH_2$-O) ; 5.43 (dd, 1H, $J^{cis}$ = 10.4, $J^{gem}$ = 1.4, $CH_2$=C) ; 5.56 (dd, 1H, $J^{trans}$ = 17.6, $J^{gem}$ = 1.4, $CH_2$=C) ; 6.15-6.23 (m, 1H, CH=C) ; 6.98 (d, 1H, *J* = 2.0, $H_{6'}$) ; 7.07 (d, 1H, *J* = 8.4) ; 7.42 (dd, 1H, *J* = 8.8, *J* = 2.4) ; 7.56 (d, 1H, *J* = 8.8) ; 7.74 (dd, 1H, *J* = 8.4, *J* = 7.6) ; 8.59 (d, 1H, *J* = 8.4) ; 8.64-8.68 (m, 2H)
RMN $^{13}C$ (100.6 MHz, $CDCl_3$) δ ppm : 31.6, 32.1, 34.6, 35.9, 70.1, 106.7, 116.0, 119.2, 123.3, 124.3, 126.4, 126.5, 128.3, 129.0, 129.4, 130.3, 132.3, 132.4, 133.5, 134.2, 144.2, 150.3, 160.3, 165.3, 165.9
Infrarouge (neat, $cm^{-1}$) : 2962, 2873, 1702, 1662, 1589, 1355, 1234
Analyse élémentaire : calculé (trouvé) pour $C_{29}H_{31}NO_3$ :

C : 78,88% (78,9%)
H : 7,08% (7,1%)
N : 3.17% (3,6%)

**1.4. *N*-(2',5'-di-*t*-butylphényl)-4-octyloxy-1,8-naphtalimide**

Le composé titre, qui correspond au dérivé **7** représenté ci-avant, est synthétisé selon un protocole identique à celui décrit ci-dessus pour la synthèse du dérivé **4** à ceci près que la réaction entre le composé **3** et l'hydroxyde de potassium a lieu dans l'octanol à 70°C pendant 16h heures. On obtient ainsi 214 mg du dérivé **7** sous la forme d'un solide jaune pâle (Rdt : 29%).

RMN $^1H$ (250 MHz, $CDCl_3$) δ ppm : 0.88 (t, 3H, *J* = 6.0, $CH_3$) ; 1.27 (s, 9H, $CH_3$) ; 1.30 (s, 9H, $CH_3$) 1.30-1.70 (m, 10H) ; 2.00 (qt, 2H *J* = 6.8, $CH_2$) ; 4.30 (t, 2H, *J* = 6.3, $CH_2$) ; 6. 99 (d, 1H, *J* = 2.1, $H_{6'}$) ; 7.06 (d, 1H, *J* = 8.3) ; 7.40-7.47 (m, 1H) ; 7.54-7.59 (m, 1H) ; 7.74 (t, 1H, J = 8.1, $H_6$) ; 8.57-8.67 (m, 3H)
RMN $^{13}C$ (62.9 MHz, $CDCl_3$) δ ppm : 14.1, 22.7, 26.1, 29.0, 29.2, 29.3, 31.2, 31.7, 31.8, 34.2, 35.5, 69.1, 105.9, 115.2, 122.8, 123.8, 125.9, 127.9, 128.6, 131.2, 131.6, 131.9, 132.5, 133.2, 133.9, 143.8, 149.9, 160.6, 164.9, 165.5.

### 1.5. *N*-(2',5'-di-*t*-butylphényl)-4-éthylsulfanyl-1,8-naphtalimide

Le composé titre, qui correspond au dérivé **8** représenté ci-avant, est synthétisé selon un protocole identique à celui décrit ci-dessus pour la synthèse du dérivé **4** à ceci près que la réaction entre le composé **3** et l'hydroxyde de potassium a lieu dans l'éthanethiol au reflux, à 60°C pendant 5 heures. On obtient ainsi 460 mg du dérivé **8** sous la forme d'un solide jaune (Rdt : 66%).

RMN $^1$H (250 MHz, $CDCl_3$) δ ppm : 1.19 (s, 9H, $CH_3$) ; 1.23 (s, 9H, $CH_3$) ; 1.41 (t, 3H, *J* = 7.4, $CH_3$) ; 3.13 (qd, 2H, *J* = 7.4, $CH_2$) ; 6.90 (d, 1H, *J* = 2.0, $H_{6'}$) ; 7.33-7.38 (m, 1H) ; 7.46-7.51 (m, 2H) ; 7.69 (t, 1H, *J* = 7.5, $H_6$) ; 8.44 (d, 1H, *J* = 7.9) ; 8.50-8.60 (m, 2H)
RMN $^{13}$C (62.9 MHz, $CDCl_3$) δ ppm : 13.6, 26.4, 31.2, 31.7, 33.9, 35.5, 115.0, 115.7, 119.5, 122.7, 123.6, 126.1, 126.6, 127.8, 128.7, 129.7, 130.3, 131.2, 132.9, 143.8, 145.5, 150.0, 165.03, 165.05.

### 1.6. *N*-(2',5'-di-*t*-butylphényl)-4-octylsulfanyl-1,8-naphtalimide

Le composé titre, qui correspond au dérivé **9** représenté ci-avant, est synthétisé selon un protocole identique à celui décrit ci-dessus pour la synthèse du dérivé **4** à ceci près que la réaction entre le composé **3** et l'hydroxyde de potassium a lieu dans l'octanethiol au reflux, à 60°C pendant 16 heures. On obtient ainsi 887 mg du dérivé **9** sous la forme d'un solide jaune (Rdt : 81%).

RMN $^1$H (250 MHz, $CDCl_3$) δ ppm : 0.81 (t, 3H, J = 6.9, $CH_3$) ; 1.20 (s, 9H, $CH_3$) ; 1.20-1.56 (m, 10H) ; 1.24 (s, 9H, $CH_3$) ; 1.76 (qt, 1H, J = 7.6, $CH_2$) ; 3.11 (t, 2H, J = 7.4, $CH_2$) ; 6.91 (d, 1H, J = 2.2, $H_{6'}$) ; 7.36 (dd, 1H, J = 8.5, J = 2.2) ; 7.48-7.52 (m, 2H) ; 7.70 (t, 1H, *J* = 8.4, $H_6$) ; 8.45 (d, 1H, *J* = 7.9) ; 8.54-8.61 (m, 2H)

RMN $^{13}$C (62.9 MHz, $CDCl_3$) δ ppm : 14.1, 22.6, 28.4, 29.0, 29.7, 31.2, 31.3, 31.7, 31.8, 32.3, 34.2, 35.5, 115.0, 115.7, 119.4, 122.6, 123.6, 126.1, 126.6, 127.8, 128.7, 129.8, 130.3, 131.1, 132.9, 143.8, 145.9, 149.9, 165.03, 165.05.

### 1.7. *N*-butyl-4-éthoxy-1,8-naphtalimide

Le composé titre, qui correspond au dérivé **12** représenté ci-avant, est synthétisé en partant du composé **1** et de la n-butylamine ou composé **10,** selon le schéma réactionnel suivant :

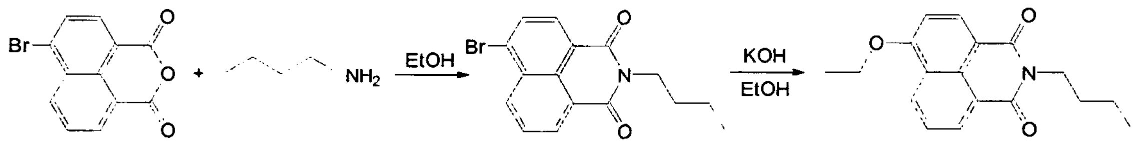


**1** **10** **11** **12**

Synthèse du N-butyl-4-bromo-1,8-naphtalimide ou composé **11**

Dans un ballon de 100 mL, on solubilise 399 mg (1,44 mmol) du composé **1** dans 20 mL d'éthanol. Puis, 154 μL (1,55 mmol) du composé **10** sont ajoutés et la solution est chauffée à 80°C pendant 8 heures. Après retour à température ambiante, le précipité formé est filtré puis lavé deux fois à l'eau. Il est séché puis recristallisé dans l'éthanol à chaud. On isole ainsi le composé **11** sous la forme de paillettes jaunes (336 mg, Rdt : 72 %).

Synthèse du dérivé **12**

Dans un ballon de 25 mL, 332 mg (1,0 mmol) du composé **11** et 97,8 mg (1,5 mmol) d'hydroxyde de potassium sont recouverts d'éthanol absolu. Le mélange est chauffé à 80°C pendant 16 heures. Après retour à température ambiante, le solvant est évaporé et le résidu directement chromatographié par colonne sur gel de silice. On obtient ainsi 115 mg du dérivé **12** sous la forme d'un solide jaune clair (Rdt : 39 %).

Les résultats des analyses de caractérisation de ce composé sont conformes à celles décrites dans la littérature.

### Exemple 2 : Propriétés des scintillateurs liquides selon l'invention

### 2.1. Propriétés physiques

Les performances de six scintillateurs liquides selon l'invention ont été testées et comparées à celles de quatre scintillateurs organiques liquides du commerce.

Les scintillateurs liquides selon l'invention sont constitués du dérivé **4** en solution à 10 g/L dans respectivement le *m*-xylène, le toluène, le mésitylène, le benzène, le p-xylène et le p-isopropylbiphényle, tandis que les scintillateurs organiques liquides du commerce sont (1) un mélange de p-terphényle et de POPOP (1,4-di-(2-(5-phényloxazolyl)) benzène) dans un rapport de 4 à 0,1 g/L dans le toluène ; (2) le NE 213 ; (3) l'Ultima Gold® AB de Perkin Elmer et (4) le BC-501A de Bicron.

L'instrumentation utilisée est celle décrite par S. Normand et al. dans Nucl. Instrum. Methods Phys. Res. A 2002, 484, 342-350. C'est la discrimination n/$\gamma$ par comparaison de charges qui est retenue.

Le photomultiplicateur est un modèle Photonis XP2020 qui fonctionne à une tension de 2 kV.

Les scintillateurs liquides sont disposés dans une cuve de (38 x 10 x 10) $mm^3$ et dont toutes les parois à l'exception de celle qui est collée au photomultiplicateur sont recouvertes d'une peinture à l'oxyde de titane.

Pour tous les tests, la chaîne de montage électronique est la même avec des réglages constants. La tension du photomultiplicateur est également gardée constante, à gain constant et la répartition entre fenêtre lente et fenêtre rapide est, elle aussi, constante.

Ont été utilisées deux sources différentes de radiations : une source de cobalt 60 de 48 kBq (qui n'émet que des gamma) pour mesurer l'intensité lumineuse produite par les scintillateurs, le temps de montée et le temps de descente des impulsions, et une source de californium 252 de 74 MBq (qui émet des neutrons et des gamma) pour déterminer la figure de mérite M (FOM) qui représente la capacité d'un scintillateur liquide à séparer le lobe des neutrons de celui des gamma, et l'angle $\theta$ qui est formé entre ces lobes.

La figure de mérite M a été calculée par une méthode améliorée de celle décrite par M. Moszyński et al. dans Nucl. Instrum. Methods Phys. Res. A 1994, 350, 226-234, à partir de l'équation :

$$\frac{\text{Séparation des pics}}{(FWHM_{\gamma} + FWHM_{n})}$$

dans laquelle FWHM représente la largeur à mi-hauteur des lobes.

Tous les scintillateurs liquides ont été soumis à un bullage à l'argon pendant au moins dix minutes.

Les résultats sont consignés dans le tableau 1 ci-après.

Ce tableau montre que les scintillateurs liquides selon l'invention présentent des propriétés spectroscopiques largement aussi intéressantes que celles des scintillateurs organiques liquides du commerce.

Tableau 1

| Scintillateur liquide | Intensité lumineuse[a] | Temps de montée (ns)[b] | Temps de descente (ns)[b] | FOM[c] | $\theta$[d] |
|---|---|---|---|---|---|
| p-terphényle/POPOP (4/0,1 g/L) dans le toluène | 100 | 2,46 | 7,29 | 0,6-1,9 (40-280) | 5,0 |
| NE213 | 85 | 2,77 | 9,05 | 0,8-1,8 (35-200) | 7,8 |
| Ultima Gold® AB | 77 | 2,55 | 9,90 | 0,7-1,3 (40-135) | 6,3 |
| BC-501A | 64 | 2,73 | 8,61 | 1,0-2,4 (35-250) | 8,3 |
| Dérivé **4** à 10 g/L dans le m-xylène | 64 | 2,93 | 14,86 | 0,6-1,6 (40-260) | 5,0 |
| Dérivé **4** à 10 g/L dans le toluène | 59 | 2,96 | 14,92 | 0,6-1,6 (35-270) | 5,1 |
| Dérivé **4** à 10 g/L dans le mésitylène | 56 | 3,02 | 13,82 | 0,4-1,2 (45-200) | 3,5 |
| Dérivé **4** à 10 g/L dans le benzène | 51 | 3,06 | 15,24 | 0,3-1,4 (50-200) | 7,0 |
| Dérivé **4** à 10 g/L dans le p-xylène | 50 | 3,01 | 15,25 | 0,6-1,4 (40-210) | 4,8 |

(suite)

| Scintillateur liquide | Intensité lumineuse[a] | Temps de montée (ns)[b] | Temps de descente (ns)[b] | FOM[c] | θ[d] |
|---|---|---|---|---|---|
| Dérivé **4** à 10 g/L dans le p-isopropylbiphényle | 33 | 3,45 | 13,16 | 0,5-0,9 (45-200) | 3,1 |

[a] rapportée à 100 pour le scintillateur de référence (POPOP)
[b] pris entre 20 et 80% de la partie de l'impulsion concernée
[c] calculé depuis l'équation $\frac{\text{Séparation des pics}}{(FWHM_\gamma + FWHM_n)}$, où FWHM représente la largeur à mi-hauteur des lobes ; est indiqué entre parenthèses, l'intervalle de mesure sur laquelle la FOM a été déterminée.
[d] angle en degrés formé entre le lobe des neutrons et celui des gamma.

Par ailleurs, l'aptitude à discriminer les neutrons rapides et les rayons gamma de scintillateurs liquides selon l'invention, préparés à partir d'un dérivé du 1,8-naphtalimide autre que le dérivé **4,** a également été testée en utilisant une instrumentation et des conditions opératoires identiques à celles décrites ci-avant.

Ces scintillateurs liquides sont respectivement constitués du dérivé **5,** du dérivé **8,** du dérivé **9** et du dérivé **12** en solution à 10 g/L dans le toluène.

La figure de mérite M (FOM) et l'angle θ formé entre le lobe des neutrons et celui des gamma obtenus pour chacun des scintillateurs liquides testés sont présentés dans le tableau 2 ci-après.

Tableau 2

| Scintillateur liquide | FOM[c] | θ[d] |
|---|---|---|
| Dérivé **5** à 10 g/L dans le toluène | 0,4-1,1 (35-150) | 5,5 |
| Dérivé **6** à 10 g/L dans le toluène | n.d. | n.d. |
| Dérivé **7** à 10 g/L dans le toluène | n.d. | n.d. |
| Dérivé **8** à 10 g/L dans le toluène | 0,3-1,2 (40-180) | 4,7 |
| Dérivé **9** à 10 g/L dans le toluène | 0,4-1,0 (64-150) | 6,7 |
| Dérivé **12** à 10 g/L dans le toluène | 0,2-0,8 (40-180) | 3,6 |

[c] calculé depuis l'équation $\frac{\text{Séparation des pics}}{(FWHM_\gamma + FWHM_n)}$, où *FWHM* représente la largeur à mi-hauteur des lobes ; est indiqué entre parenthèses, l'intervalle de mesure sur laquelle la FOM a été déterminée ;
[d] angle en degrés formé entre le lobe des neutrons et celui des gamma ;
n.d. : non déterminé en raison d'une valeur trop faible.

### 2.2. Insensibilité à l'oxygène dissous

La sensibilité à l'oxygène dissous d'un scintillateur liquide selon l'invention, constitué du dérivé **4** en solution à 10 g/L dans le toluène, a été testée et comparée à celle des scintillateurs liquides NE213, BC-501A et Ultima Gold® AB.

Pour ce faire, l'aptitude à discriminer les neutrons rapides et les rayons gamma a été testée pour chaque scintillateur liquide après saturation d'argon et après saturation d'oxygène, en utilisant une instrumentation et des conditions opératoires identiques à celles décrites ci-avant.

La figure de mérite M (FOM), la moyenne de la figure de mérite M prise dans les valeurs de charge totale comprise entre 50 et 100 pC (<FOM>) et l'angle θ formé entre le lobe des neutrons et celui des gamma obtenus pour chacun des scintillateurs liquides testés sont présentés dans le tableau 3 ci-après.

Tableau 3

| Scintillateur liquide | FOM | <FOM> | θ |
|---|---|---|---|
| Dérivé **4** à 10 g/L dans le toluène saturé en argon | 0,6-1,6 (35-270) | 0,93 | 5,1 |
| NE213 saturé en argon | 0,8-1,8 | 1,39 | 7,8 |
| BC-501A saturé en argon | 1,0-2,4 (35-250) | 1,66 | 8,3 |
| Ultima Gold AB saturé en argon | 0,7-1,3 (40-135) | 0,91 | 6,3 |
| Dérivé **4** à 10 g/L dans le toluène saturé en $O_2$ | 0,5-1,8 (35-250) | 1,00 | 5,5 |
| NE213 saturé en $O_2$. | 0,3-1,5 (35-220) | 0,97 | 4,1 |
| BC-501A saturé en $O_2$ | (35-240) | 1,12 | 4,1 |
| Ultima Gold AB saturé en $O_2$ | 0,3-1,2 (40-200) | 0,73 | 4,4 |

La discrimination n/γ telle qu'obtenue, après saturation d'argon et saturation d'oxygène, pour les différents scintillateurs liquides testés est également illustrée sur les figures 2 à 9.

Le tableau 3 et ces figures montrent que le scintillateur liquide selon l'invention conserve ses performances en termes de discrimination n/γ lorsqu'il est saturé en oxygène contrairement aux scintillateurs organiques liquides du commerce pour lesquels on peut notamment observer que l'angle θ formé entre le lobe des neutrons et celui des gamma diminue d'un facteur proche de 2 après saturation en oxygène.

Par ailleurs, des tests ont permis de confirmer que le scintillateur liquide selon l'invention ne nécessite aucune précaution particulière pour son conditionnement et son stockage. Il peut être ainsi conservé sous atmosphère d'air et à la lumière du jour sans que ses propriétés de discrimination soient affectées, même après plusieurs mois.

## Revendications

**1.** Utilisation d'un dérivé du 1,8-naphtalimide répondant à la formule générale (I) ci-après :

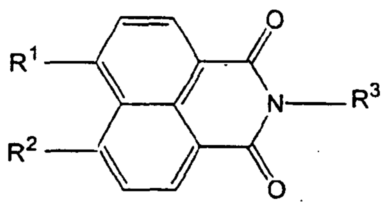

(I)

dans laquelle :

- $R^1$ représente un groupe choisi parmi :

  - les groupes -OR' et -SR' où R' représente un groupe hydrocarboné linéaire ou ramifié, saturé ou insaturé, en $C_1$ à $C_{20}$, ou un groupe hydrocarboné cyclique saturé en $C_3$ à $C_{10}$, ou encore un groupe aryle ou hétéroaryle ; et
  - les groupes -NR'R" où R' est tel que précédemment défini, tandis que R" représente soit un atome d'hydrogène, soit un groupe hydrocarboné linéaire ou ramifié, saturé ou insaturé, en $C_1$ à $C_{20}$, soit un groupe hydrocarboné cyclique saturé en $C_3$ à $C_{10}$, soit encore un groupe aryle ou hétéroaryle ;

- $R^2$ représente un atome d'hydrogène ou un groupe identique ou différent de $R^1$, choisi parmi :

  - les groupes -OR' et -SR' où R' représente un groupe hydrocarboné linéaire ou ramifié, saturé ou insaturé, en $C_1$ à $C_{20}$, ou un groupe hydrocarboné cyclique saturé en $C_3$ à $C_{10}$, ou encore un groupe aryle ou hétéroaryle ; et

- les groupes -NR'R" où R' est tel que précédemment défini, tandis que R" représente soit un atome d'hydrogène, soit un groupe hydrocarboné linéaire ou ramifié, saturé ou insaturé, en $C_1$ à $C_{20}$, soit un groupe hydrocarboné cyclique saturé en $C_3$ à $C_{10}$, soit encore un groupe aryle ou hétéroaryle ; tandis que

● $R^3$ représente :

- un groupe acyle, ou
- un groupe hydrocarboné linéaire ou ramifié, saturé ou insaturé, en $C_1$ à $C_{20}$, ou
- un groupe hydrocarboné cyclique saturé en $C_3$ à $C_{10}$, ou
- un groupe hétérocyclique saturé en $C_3$ à $C_{10}$, ou encore
- un groupe aryle ou hétéroaryle ;

ou de l'un de ses sels en tant qu'agent de scintillation pour discriminer les neutrons rapides des rayons gamma

**2.** Utilisation selon la revendication 1, dans laquelle le dérivé du 1,8-naphtalimide présente une longueur d'onde d'émission comprise entre 350 et 550 nm et, mieux encore, entre 380 et 470 nm.

**3.** Utilisation selon la revendication 1 ou la revendication 2, dans laquelle le dérivé du 1,8-naphtalimide répond à la formule générale (I) dans laquelle $R^1$ représente un groupe -OR' ou -SR' où R' représente un groupe hydrocarboné linéaire ou ramifié, saturé ou insaturé, en $C_1$ à $C_{20}$, ou un groupe hydrocarboné cyclique saturé en $C_3$ à $C_{10}$, ou encore un groupe aryle ou hétéroaryle, tandis que $R^2$ représente un atome d'hydrogène.

**4.** Utilisation selon la revendication 3, dans laquelle le dérivé du 1,8-naphtalimide répond à la formule générale (I) dans laquelle $R^1$ représente un groupe alcoxy, alcényloxy ou alkylsulfanyle en $C_1$ à $C_{20}$ et, mieux encore, en $C_1$ à $C_{10}$.

**5.** Utilisation selon l'une quelconque des revendications précédentes, dans laquelle le dérivé du 1,8-naphtalimide répond à la formule générale (I) dans laquelle $R^3$ représente un groupe aryle ou hétéroaryle substitué en positions 2 et 5 par rapport à l'atome d'azote du noyau naphtalimide par un groupe alkyle ramifié en $C_3$ à $C_6$.

**6.** Utilisation selon la revendication 5, dans laquelle le dérivé du 1,8-naphtalimide répond à la formule générale (I) dans laquelle $R^3$ représente un groupe di-*t*-butylphényle.

**7.** Utilisation selon l'une quelconque des revendications précédentes, dans laquelle le dérivé du 1,8-naphtalimide répond à l'une quelconque des formules particulières ci-après :

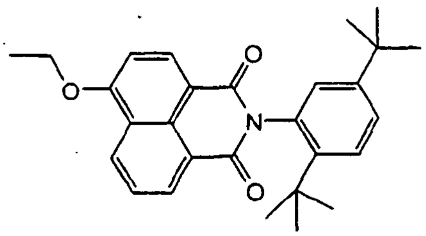

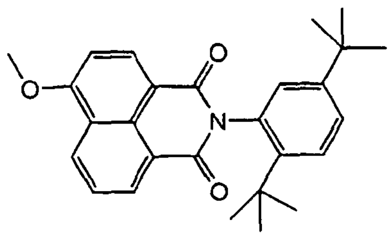

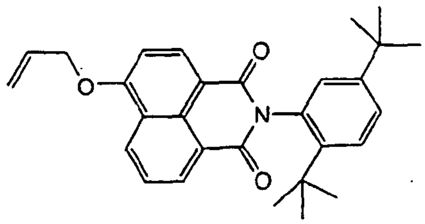

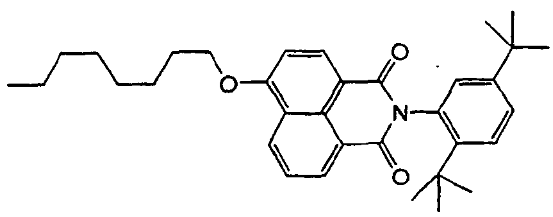

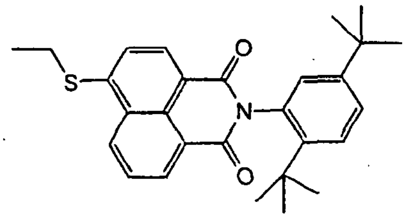

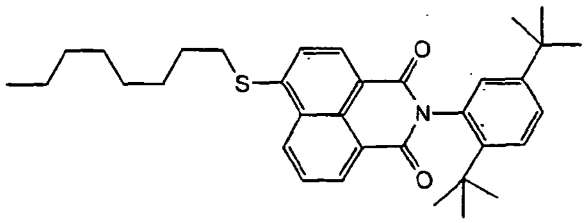

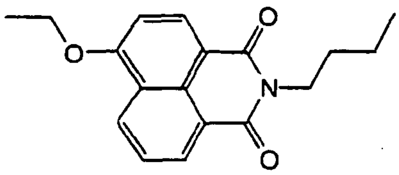

8. Utilisation selon l'une quelconque des revendications précédentes, dans laquelle le dérivé du 1,8-naphtalimide est incorporé dans une matrice solide.

9. Utilisation selon l'une quelconque des revendications 1 à 7, dans laquelle le dérivé du 1,8-naphtalimide est en solution dans un solvant.

10. Utilisation selon la revendication 9, dans laquelle le dérivé du 1,8-naphtalimide est présent dans le solvant à une concentration au moins égale à 3 g/L et, de préférence, comprise entre 8 et 12 g/L.

11. Scintillateur liquide qui comprend un dérivé du 1,8-naphtalimide répondant à la formule générale (I) ci-après :

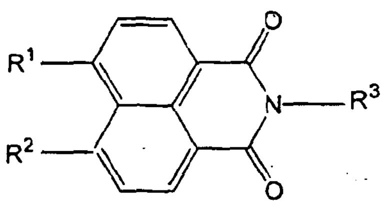

(I)

dans laquelle :

- $R^1$ représente un groupe choisi parmi :

  - les groupes -OR' et -SR' où R' représente un groupe hydrocarboné linéaire ou ramifié, saturé ou insaturé, en $C_1$ à $C_{20}$, ou un groupe hydrocarboné cyclique saturé en $C_3$ à $C_{10}$, ou encore un groupe aryle ou hétéroaryle ; et
  - les groupes -NR'R" où R' est tel que précédemment défini, tandis que R" représente soit un atome d'hydrogène, soit un groupe hydrocarboné linéaire ou ramifié, saturé ou insaturé, en $C_1$ à $C_{20}$, soit un groupe hydrocarboné cyclique saturé en $C_3$ à $C_{10}$, soit encore un groupe aryle ou hétéroaryle ;

- $R^2$ représente un atome d'hydrogène ou un groupe identique ou différent de $R^1$, choisi parmi :

  - les groupes -OR' et -SR' où R' représente un groupe hydrocarboné linéaire ou ramifié, saturé ou insaturé, en $C_1$ à $C_{20}$, ou un groupe hydrocarboné cyclique saturé en $C_3$ à $C_{10}$, ou encore un groupe aryle ou hétéroaryle ; et
  - les groupes -NR'R" où R' est tel que précédemment défini, tandis que R" représente soit un atome d'hydrogène, soit un groupe hydrocarboné linéaire ou ramifié, saturé ou insaturé, en $C_1$ à $C_{20}$, soit un groupe hydrocarboné cyclique saturé en $C_3$ à $C_{10}$, soit encore un groupe aryle ou hétéroaryle ; tandis que

- $R^3$ représente :

  - un groupe acyle, ou

- un groupe hydrocarboné linéaire ou ramifié, saturé ou insaturé, en $C_1$ à $C_{20}$, ou
- un groupe hydrocarboné cyclique saturé en $C_3$ à $C_{10}$, ou
- un groupe hétérocyclique saturé en $C_3$ à $C_{10}$,, ou encore
- un groupe aryle ou hétéroaryle ;

ou l'un de ses sels, en solution dans un solvant.

**12.** Scintillateur liquide selon la revendication 11, dans lequel le dérivé du 1,8-naphtalimide présente une longueur d'onde d'émission comprise entre 350 et 550 nm et, mieux encore, entre 380 et 470 nm.

**13.** Scintillateur liquide selon la revendication 11 ou la revendication 12, dans lequel le dérivé du 1,8-naphtalimide répond à la formule générale (I) dans laquelle $R^1$ représente un groupe -OR' ou -SR' où R' représente un groupe hydrocarboné linéaire ou ramifié, saturé ou insaturé, en $C_1$ à $C_{20}$, ou un groupe hydrocarboné cyclique saturé en $C_3$ à $C_{10}$, ou encore un groupe aryle ou hétéroaryle, tandis que $R^2$ représente un atome d'hydrogène.

**14.** Scintillateur liquide selon la revendication 13, dans lequel le dérivé du 1,8-naphtalimide répond à la formule générale (I) dans laquelle $R^1$ représente un groupe alcoxy, alcényloxy ou alkylsulfanyle en $C_1$ à $C_{20}$ et, mieux encore, en $C_1$ à $C_{10}$.

**15.** Scintillateur liquide selon l'une quelconque des revendications 11 à 14, dans lequel le dérivé du 1,8-naphtalimide répond à la formule générale (I) dans laquelle $R^3$ représente un groupe aryle ou hétéroaryle substitué en positions 2 et 5 par rapport à l'atome d'azote du noyau naphtalimide par un groupe alkyle ramifié en $C_3$ à $C_6$.

**16.** Scintillateur liquide selon la revendication 15, dans lequel le dérivé du 1,8-naphtalimide répond à la formule générale (I) dans laquelle $R^3$ représente un groupe di-*t*-butylphényle.

**17.** Scintillateur liquide selon l'une quelconque des revendications 11 à 16, dans lequel le dérivé du 1,8-naphtalimide répond à l'une quelconque des formules particulières ci-après :

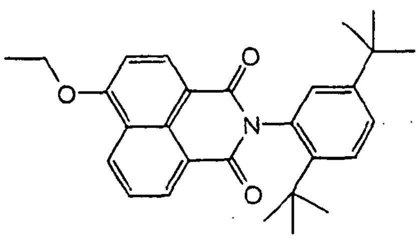
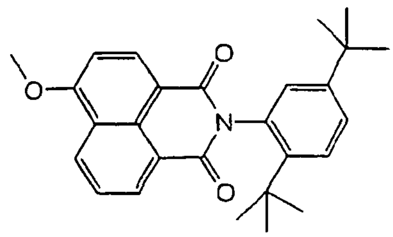

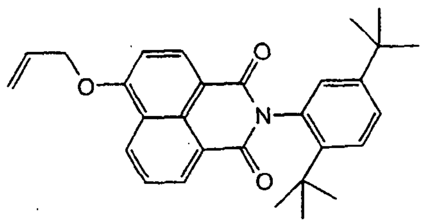
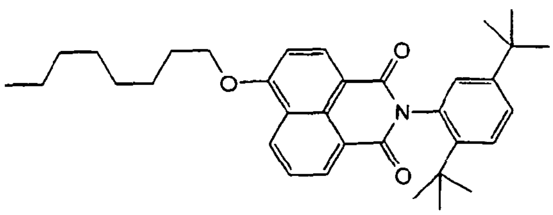

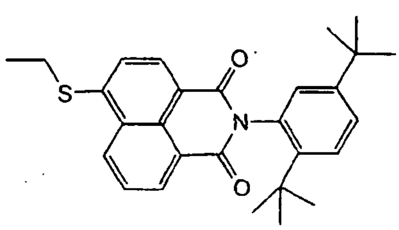
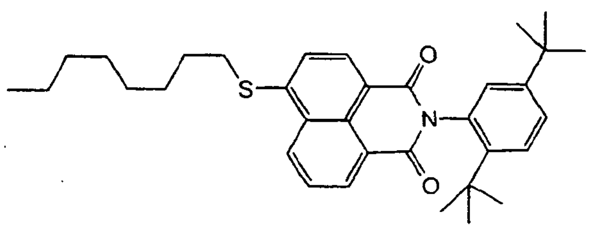

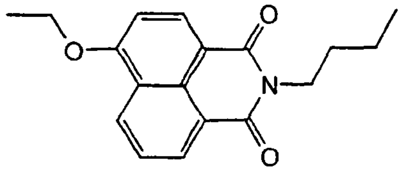

18. Scintillateur liquide selon l'une quelconque des revendications 11 à 17, dans lequel le dérivé du 1,8-naphtalimide est présent à une concentration au moins égale à 3 g/L et, de préférence, comprise entre 8 et 12 g/L.

19. Dérivé du 1,8-naphtalimide qui répond à la formule générale (I) ci-après :

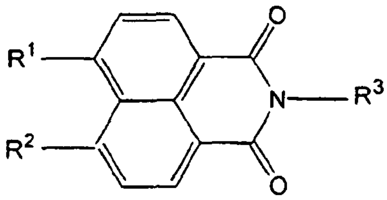

(I)

dans laquelle :

- $R^1$ représente un groupe choisi parmi les groupes -OR' et -SR' où R' représente un groupe hydrocarboné linéaire ou ramifié, saturé ou insaturé, en $C_1$ à $C_{20}$, ou un groupe hydrocarboné cyclique saturé en $C_3$ à $C_{10}$, ou encore un groupe aryle ou hétéroaryle ;
- $R^2$ représente un atome d'hydrogène ou un groupe également choisi parmi les groupes -OR' et -SR' où R' représente un groupe hydrocarboné linéaire ou ramifié, saturé ou insaturé, en $C_1$ à $C_{20}$, ou un groupe hydrocarboné cyclique saturé en $C_3$ à $C_{10}$, ou encore un groupe aryle ou hétéroaryle; tandis que
- $R^3$ représente un groupe aryle ou hétéroaryle substitué en positions 2 et 5 par rapport à l'atome d'azote du noyau naphtalimide par un groupe alkyle ramifié en $C_3$ à $C_6$ ;

et ses sels.

20. Dérivé du 1,8-naphtalimide selon la revendication 19, qui répond à la formule générale (I) dans laquelle $R^2$ représente un atome d'hydrogène.

21. Dérivé du 1,8-naphtalimide selon la revendication 19 ou la revendication 20, qui répond à la formule générale (I) dans laquelle $R^1$ représente un groupe alcoxy, alcényloxy ou alkylsulfanyle en $C_1$ à $C_{20}$ et, mieux encore, en $C_1$ à $C_{10}$.

22. Dérivé du 1,8-naphtalimide selon la revendication 19, qui répond à formule générale (I) dans laquelle $R^3$ représente un groupe di-t-butylphényle.

23. Dérivé du 1,8-naphtalimide selon l'une quelconque des revendications 19 à 22, qui répond à l'une quelconque des formules particulières ci-après :

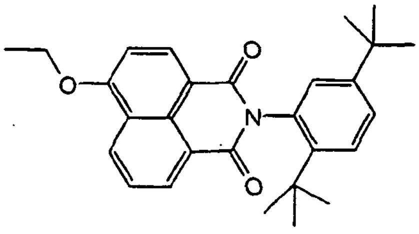

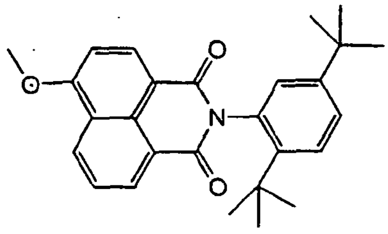

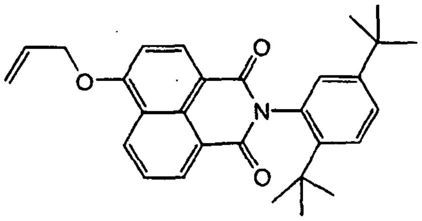

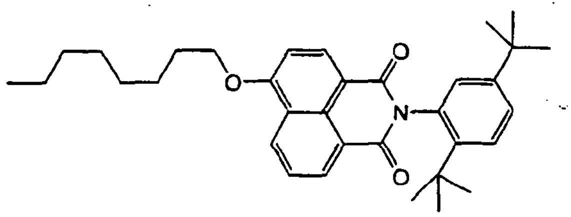

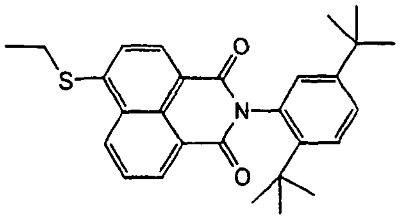

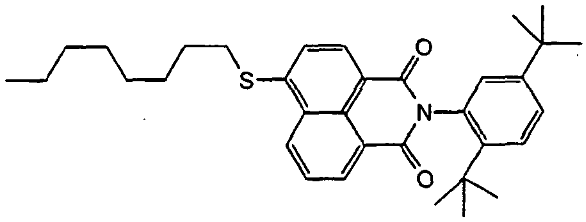

**24.** Dérivé du 1,8-naphtalimide selon la revendication 23, qui répond à la formule particulière ci-après :

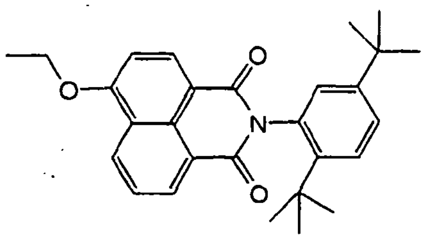

**Patentansprüche**

**1.** Verwendung eines 1,8-Naphthalimid-Derivats, welches der folgenden allgemeinen Formel (I) entspricht:

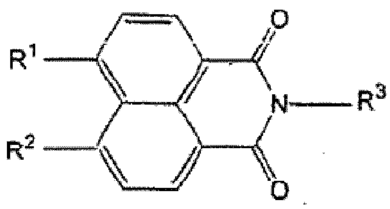

(I)

wobei:

- $R^1$ eine Gruppe ist, ausgewählt aus:

  - den Gruppen -OR' und -SR', wobei R' eine lineare oder verzweigte, gesättigte oder ungesättigte $C_1$ - $C_{20}$ Kohlenwasserstoffgruppe, oder eine cyclische gesättigte $C_3$ - $C_{10}$ Kohlenwasserstoffgruppe, oder auch eine Aryl- oder Heteroarylgruppe, darstellt; und
  - den Gruppen -NR'R", wobei R' wie zuvor definiert ist, während R" entweder ein Wasserstoffatom, oder eine lineare oder verzweigte, gesättigte oder ungesättigte $C_1$-$C_{20}$ Kohlenwasserstoffgruppe, oder eine gesättigte cyclische $C_3$ - $C_{10}$ Kohlenwasserstoffgruppe, oder auch eine Aryl- oder Heteroarylgruppe darstellt;

- $R^2$ ein Wasserstoffatom oder Gruppe ist, die identisch oder unterschiedlich zu $R^1$ ist, ausgewählt aus:

- den Gruppen -OR' und -SR', wobei R' eine lineare oder verzweigte, gesättigte oder ungesättigte $C_1$ - $C_{20}$ Kohlenwasserstoffgruppe, oder eine cyclische gesättigte $C_3$ - $C_{10}$ Kohlenwasserstoffgruppe, oder auch eine Aryl- oder Heteroarylgruppe darstellt; und
- den Gruppen -NR'R", wobei R' wie zuvor definiert ist, während R" entweder ein Wasserstoffatom, oder eine lineare oder verzweigte, gesättigte oder ungesättigte $C_1$ - $C_{20}$ Kohlenwasserstoffgruppe, oder eine gesättigte cyclische $C_3$ - $C_{10}$ Kohlenwasserstoffgruppe, oder auch eine Aryl- oder Heteroarylgruppe, darstellt; während

● $R^3$ :

- eine Acylgruppe, oder
- eine lineare oder verzweigte, gesättigte oder ungesättigte $C_1$ - $C_{20}$ Kohlenwasserstoffgruppe, oder
- eine gesättigte cyclische $C_3$ - $C_{10}$ Kohlenwasserstoffgruppe, oder
- eine heterocyclische gesättigte $C_3$ - $C_{10}$ Gruppe, oder auch
- eine Aryl- oder Heteroarylgruppe darstellt;

oder eines Salzes davon als Szintillationsmittel, um schnelle Neutronen von Gammastrahlen zu unterscheiden.

**2.** Verwendung nach Anspruch 1, wobei das 1,8-Naphthalimid-Derivat eine Emissionswellenlänge zwischen 350 und 550 nm und vorzugsweise zwischen 380 und 470 nm aufweist.

**3.** Verwendung nach Anspruch 1 oder Anspruch 2, wobei das 1,8-Naphthalimid-Derivat der allgemeinen Formel (I) entspricht, wobei $R^1$ eine -OR' oder -SR' Gruppe darstellt, wobei R' eine lineare oder verzweigte, gesättigte oder ungesättigte $C_1$ - $C_{20}$ Kohlenwasserstoffgruppe, oder eine cyclische gesättigte $C_3$ - $C_{10}$ Kohlenwasserstoffgruppe, oder auch eine Aryl- oder Heteroarylgruppe darstellt, während $R^2$ ein Wasserstoffatom darstellt.

**4.** Verwendung nach Anspruch 3, wobei das 1,8-Naphthalimid-Derivat der allgemeinen Formel (I) entspricht, wobei $R^1$ eine $C_1$ - $C_{20}$, vorzugsweise eine $C_1$ - $C_{10}$ Alkoxy-, Alkenyloxy- oder Alkylsulfanylgruppe darstellt.

**5.** Verwendung nach einem der vorhergehenden Ansprüche, wobei das 1,8-Naphthalimid-Derivat der allgemeinen Formel (I) entspricht, wobei $R^3$ eine Aryl- oder Heteroarylgruppe darstellt, welche an Positionen 2 und 5 in Bezug auf das Stickstoffatom des Naphthalimidkerns durch eine verzweigte $C_3$ - $C_6$ Alkylgruppe substituiert ist.

**6.** Verwendung nach Anspruch 5, wobei das 1,8-Naphthalimid-Derivat der allgemeinen Formel (I) entspricht, wobei $R^3$ eine Di-*t*-butylphenylgruppe darstellt.

**7.** Verwendung nach einem der vorhergehenden Ansprüche, wobei das 1,8-Naphthalimid-Derivat einer der folgenden spezifischen Formeln entspricht:

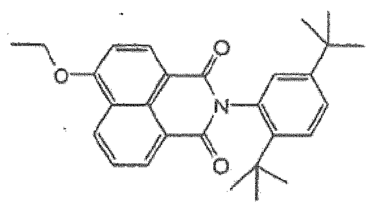

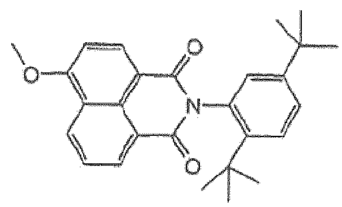

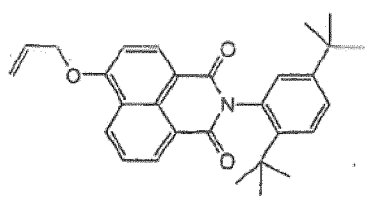

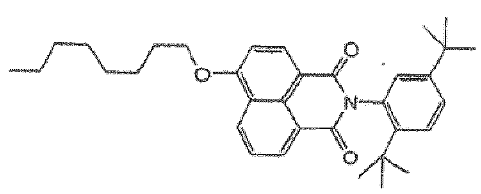

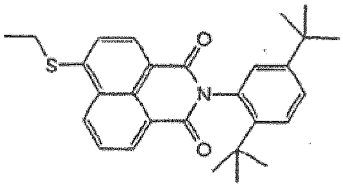

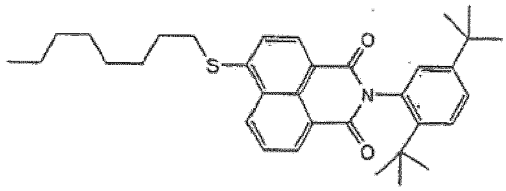

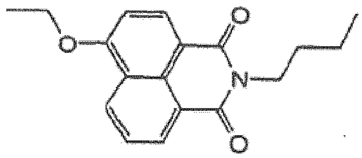

**8.** Verwendung nach einem der vorhergehenden Ansprüche, wobei das 1,8-Naphthalimid-Derivat in eine feste Matrix eingebettet ist.

**9.** Verwendung nach einem der Ansprüche 1 bis 7, wobei das 1,8-Naphthalimid-Derivat in einem Lösungsmittel gelöst ist.

**10.** Verwendung nach Anspruch 9, wobei das 1,8-Naphthalimid-Derivat in dem Lösungsmittel in einer Konzentration von mindestens 3 g/L, und vorzugsweise, zwischen 8 und 12 g/L vorliegt.

**11.** Flüssiges Szintillationsmittel umfassend ein 1,8-Naphthalimid-Derivat, welches der folgenden allgemeinen Formel (I) entspricht:

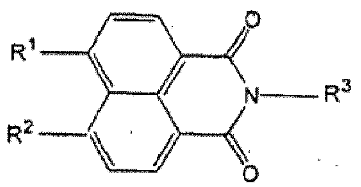

(I)

wobei:

- $R^1$ eine Gruppe darstellt, ausgewählt aus:

  - den Gruppen -OR' und -SR', wobei R' eine lineare oder verzweigte, gesättigte oder ungesättigte $C_1$ - $C_{20}$ Kohlenwasserstoffgruppe, oder eine cyclische gesättigte $C_3$ - $C_{10}$ Kohlenwasserstoffgruppe, oder auch eine Aryl- oder Heteroarylgruppe, darstellt; und
  - den Gruppen -NR'R", wobei R' wie zuvor definiert ist, während R" entweder ein Wasserstoffatom, oder eine lineare oder verzweigte, gesättigte oder ungesättigte $C_1$ - $C_{20}$ Kohlenwasserstoffgruppe, oder eine gesättigte cyclische $C_3$ - $C_{10}$ Kohlenwasserstoffgruppe, oder auch eine Aryl- oder Heteroarylgruppe darstellt;

- $R^2$ ein Wasserstoffatom oder eine Gruppe ist, die identisch oder unterschiedlich zu $R^1$ ist, ausgewählt aus:

  - den Gruppen -OR' und -SR', wobei R' eine lineare oder verzweigte, gesättigte oder ungesättigte $C_1$ - $C_{20}$ Kohlenwasserstoffgruppe, oder eine cyclische gesättigte $C_3$ - $C_{10}$ Kohlenwasserstoffgruppe, oder auch eine Aryl- oder Heteroarylgruppe darstellt; und
  - den Gruppen -NR'R", wobei R' wie zuvor definiert ist, während R" entweder ein Wasserstoffatom, oder eine lineare oder verzweigte, gesättigte oder ungesättigte $C_1$ - $C_{20}$ Kohlenwasserstoffgruppe, oder eine gesättigte cyclische $C_3$ - $C_{10}$ Kohlenwasserstoffgruppe, oder auch eine Aryl- oder Heteroarylgruppe, darstellt; während

- $R^3$:

- eine Acylgruppe, oder
- eine lineare oder verzweigte, gesättigte oder ungesättigte $C_1$- $C_{20}$ Kohlenwasserstoffgruppe, oder
- eine gesättigte cyclische $C_3$ - $C_{10}$ Kohlenwasserstoffgruppe, oder
- eine heterocyclische gesättigte $C_3$-$C_{10}$ Gruppe, oder auch
- eine Aryl- oder Heteroarylgruppe darstellt;

oder ein Salz davon, welches in einem Lösungsmittel gelöst ist.

**12.** Flüssiges Szintillationsmittel nach Anspruch 11, wobei das 1,8-Naphthalimid-Derivat eine Emissionswellenlänge zwischen 350 und 550 nm und vorzugsweise zwischen 380 und 470 nm aufweist.

**13.** Flüssiges Szintillationsmittel nach Anspruch 11 oder Anspruch 12, wobei das 1,8-Naphthalimid-Derivat der allgemeinen Formel (I) entspricht, wobei, $R^1$ eine -OR' oder -SR' Gruppe darstellt, wobei R' eine lineare oder verzweigte, gesättigte oder ungesättigte $C_1$ - $C_{20}$ Kohlenwasserstoffgruppe, oder eine cyclische gesättigte $C_3$ - $C_{10}$ Kohlenwasserstoffgruppe, oder auch eine Aryl- oder Heteroarylgruppe darstellt, während $R^2$ ein Wasserstoffatom darstellt.

**14.** Flüssiges Szintillationsmittel nach Anspruch 13, wobei das 1,8-Naphthalimid-Derivat der allgemeinen Formel (I) entspricht, wobei $R^1$ eine $C_1$ - $C_{20}$, vorzugsweise eine $C_1$ - $C_{10}$ Alkoxy-, Alkenyloxy- oder Alkylsulfanylgruppe darstellt.

**15.** Flüssiges Szintillationsmittel nach einem der Ansprüche 11 bis 14, wobei das 1,8-Naphthalimid-Derivat der allgemeinen Formel (I) entspricht, wobei $R^3$ eine Aryl- oder Heteroarylgruppe darstellt, welche an Positionen 2 und 5 in Bezug auf das Stickstoffatom des Naphthalimidkerns durch eine verzweigte $C_3$ - $C_6$ Alkylgruppe substituiert ist.

**16.** Flüssiges Szintillationsmittel nach Anspruch 15, wobei das 1,8-Naphthalimid-Derivat der allgemeinen Formel (I) entspricht, wobei $R^3$ eine Di-*t*-butylphenylgruppe darstellt.

**17.** Flüssiges Szintillationsmittel nach einem der Ansprüche 11 bis 16, wobei das 1,8-Naptalimid-Derivat eine der folgenden spezifischen Formeln entspricht:

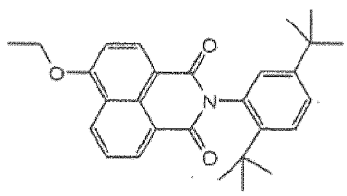
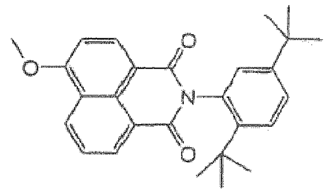

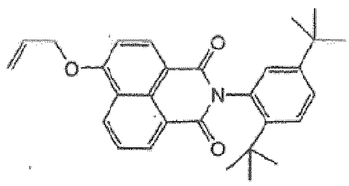
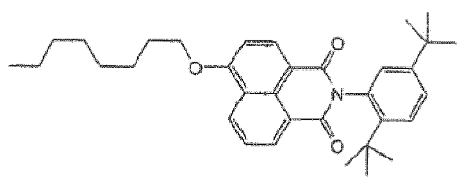

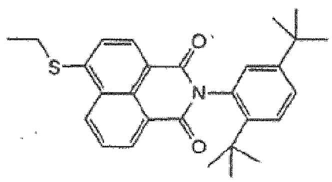
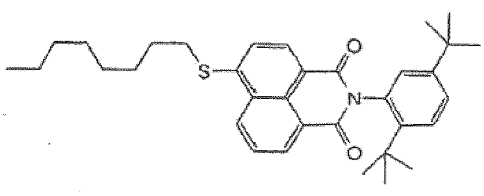

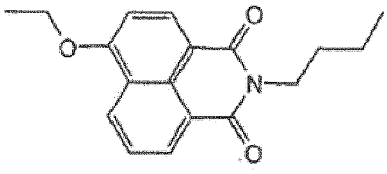

**18.** Flüssiges Szintillationsmittel nach einem der Ansprüche 11 bis 17, wobei das 1,8-Naphthalimid-Derivat in einer Konzentration von mindestens 3 g/L, und vorzugsweise zwischen 8 und 12 g/L vorliegt.

**19.** 1,8-Naphthalimid-Derivat, welches der folgenden allgemeinen Formel (I) entspricht:

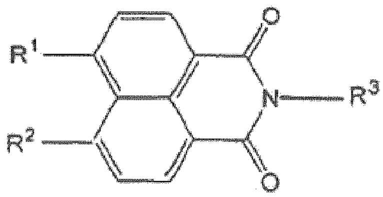

(I)

wobei:

- $R^1$ eine Gruppe darstellt, welche aus den Gruppen -OR' und -SR' ausgewählt ist, wobei R' eine lineare oder verzweigte, gesättigte oder ungesättigte $C_1$ - $C_{20}$ Kohlenwasserstoffgruppe, oder eine cyclische gesättigte $C_3$ - $C_{10}$ Kohlenwasserstoffgruppe, oder auch eine Aryl- oder Heteroarylgruppe, darstellt;
- $R^2$ ein Wasserstoffatom oder eine Gruppe ist, welche auch aus den Gruppe -OR' und -SR' ausgewählt ist, wobei R' eine lineare oder verzweigte, gesättigte oder ungesättigte $C_1$ - $C_{20}$ Kohlenwasserstoffgruppe, oder eine cyclische gesättigte $C_3$ - $C_{10}$ Kohlenwasserstoffgruppe, oder auch eine Aryl- oder Heteroarylgruppe darstellt; während
- $R^3$ eine Aryl- oder Heteroarylgruppe darstellt, welche an Positionen 2 und 5 in Bezug auf das Stickstoffatom des Naphthalimidkerns durch eine verzweigte $C_3$ - $C_6$ Alkylgruppe substituiert ist, und seine Salze.

**20.** 1,8-Naphthalimid-Derivat nach Anspruch 19, welches der allgemeinen Formel (I) entspricht, wobei $R^2$ ein Wasserstoffatom darstellt.

**21.** 1,8-Naphthalimid-Derivat nach Anspruch 19 oder Anspruch 20, welches der allgemeinen Formel (I) entspricht, wobei $R^1$ eine $C_1$ - $C_{20}$, vorzugsweise eine $C_1$ - $C_{10}$ Alkoxy-, Alkenyloxy- oder Alkylsulfanylgruppe darstellt.

**22.** 1,8-Naphthalimid-Derivat nach Anspruch 19, welches der allgemeinen Formel (I) entspricht, wobei $R^3$ eine Di-*t*-butylphenylgruppe darstellt.

**23.** 1,8-Naphthalimid-Derivat nach Anspruch 19 bis Anspruch 22, welches einer der folgenden spezifischen Formeln entspricht:

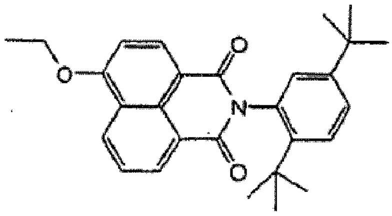

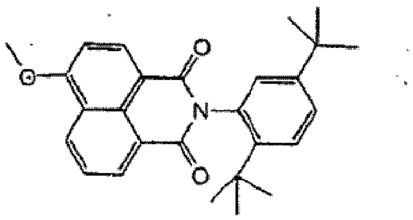

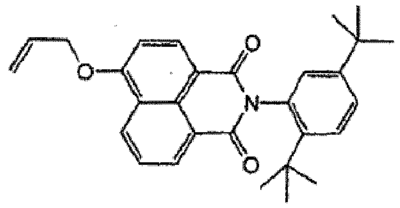

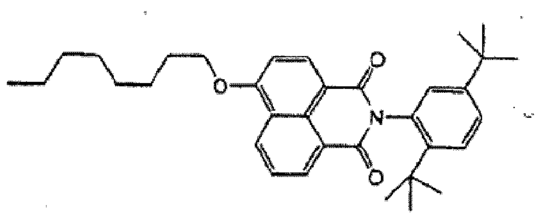

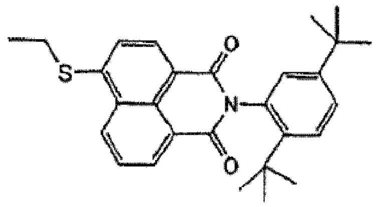

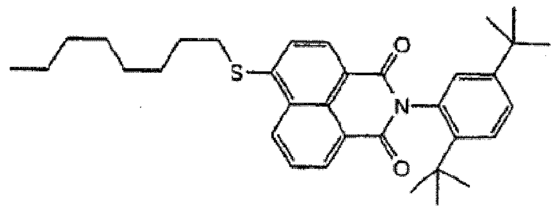

**24.** 1,8-Naphthalimid-Derivat nach Anspruch 23, welches der folgenden spezifischen Formel entspricht:

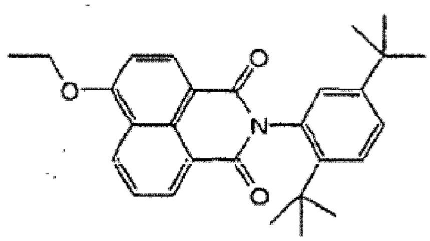

## Claims

**1.** Use of a 1,8-naphthalimide derivative corresponding to the following general formula (I):

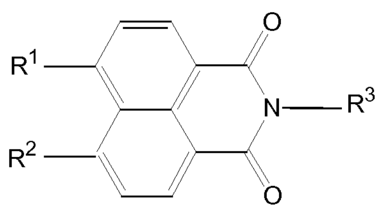

(I)

in which:

- $R^1$ represents a group chosen from:

  - -OR' and -SR' groups, where R' represents a saturated or unsaturated linear or branched $C_1$ to $C_{20}$ hydrocarbon group, or a saturated cyclic $C_3$ to $C_{10}$ hydrocarbon group, or still an aryl or heteroaryl group; and
  - -NR'R" groups, where R' is as defined above, while R" represents either a hydrogen atom, or a saturated or unsaturated and linear or branched $C_1$ to $C_{20}$ hydrocarbon group, or a saturated cyclic $C_3$ to $C_{10}$ hydrocarbon group, or still an aryl or heteroaryl group;

- $R^2$ represents a hydrogen atom or a group identical to or different from $R^1$, chosen from:

- -OR' and -SR' groups, where R' represents a saturated or unsaturated linear or branched $C_1$ to $C_{20}$ hydrocarbon group, or a saturated cyclic $C_3$ to $C_{10}$ hydrocarbon group, or still an aryl or heteroaryl group; and
- -NR'R" groups, where R' is as defined above, while R" represents either a hydrogen atom, or a saturated or unsaturated and linear or branched $C_1$ to $C_{20}$ hydrocarbon group, or a saturated cyclic $C_3$ to $C_{10}$ hydrocarbon group, or still an aryl or heteroaryl group; while

● $R^3$ represents:

- an acyl group, or
- a saturated or unsaturated and linear or branched $C_1$ to $C_{20}$ hydrocarbon group, or
- a saturated cyclic $C_3$ to $C_{10}$ hydrocarbon group, or
- a saturated $C_3$ to $C_{10}$ heterocyclic group, or still
- an aryl or heteroaryl group;

or of one of its salts, as scintillation agent for discriminating between fast neutrons and gamma rays.

2. Use according to claim 1, in which the 1,8-naphthalimide derivative exhibits an emission wavelength of between 350 and 550 nm and better still between 380 and 470 nm.

3. Use according to claim 1 or claim 2, in which the 1,8-naphthalimide derivative corresponds to the general formula (I) in which $R^1$ represents an -OR' or -SR' group, where R' represents a saturated or unsaturated and linear or branched $C_1$ to $C_{20}$ hydrocarbon group, or a saturated cyclic $C_3$ to $C_{10}$ hydrocarbon group, or still an aryl or heteroaryl group, while $R^2$ represents a hydrogen atom.

4. Use according to claim 3, in which the 1,8-naphthalimide derivative corresponds to the general formula (I) in which $R^1$ represents a $C_1$ to $C_{20}$ and better still $C_1$ to $C_{10}$ alkoxy, alkenyloxy or alkylsulphanyl group.

5. Use according to any one of the preceding claims, in which the 1,8-naphthalimide derivative corresponds to the general formula (I) in which $R^3$ represents an aryl or heteroaryl group substituted in the 2 and 5 positions, with respect to the nitrogen atom of the naphthalimide ring system, by a branched $C_3$ to $C_6$ alkyl group.

6. Use according to claim 5, in which the 1,8-naphthalimide derivative corresponds to the general formula (I) in which $R^3$ represents a di(t-butyl)phenyl group.

7. Use according to any one of the preceding claims, in which the 1,8-naphthalimide derivative corresponds to any one of the specific formulae below:

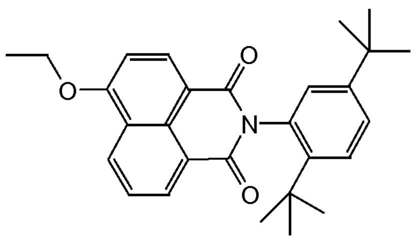

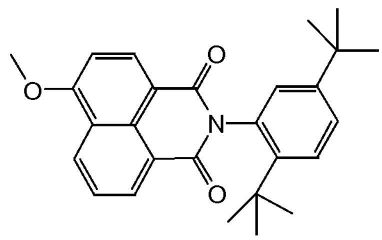

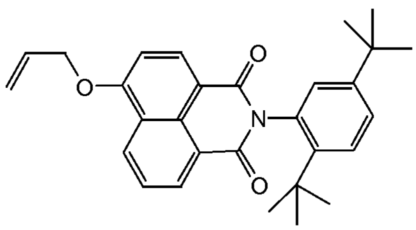

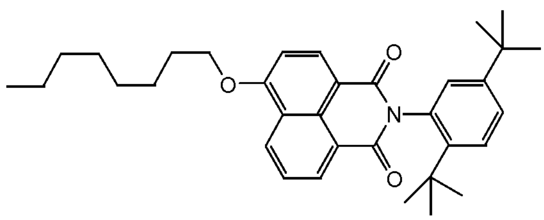

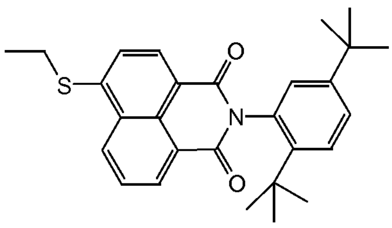

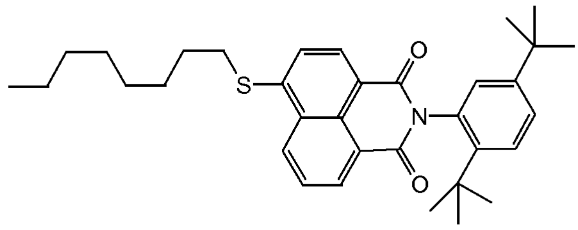

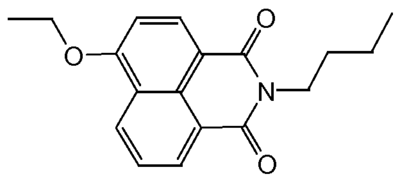

**8.** Use according to any one of the preceding claims, in which the 1,8-naphthalimide derivative is incorporated in a solid matrix.

**9.** Use according to any one of Claims 1 to 7, in which the 1,8-naphthalimide derivative is in solution in a solvent.

**10.** Use according to claim 9, in which the 1,8-naphthalimide derivative is present in the solvent at a concentration at least equal to 3 g/l and preferably of between 8 and 12 g/l.

**11.** Liquid scintillator which comprises a 1,8-naphthalimide derivative corresponding to the general formula (I) below:

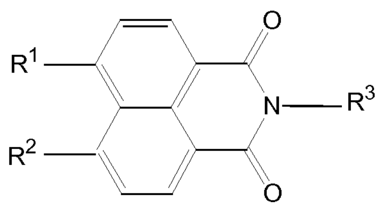

(I)

in which:

- $R^1$ represents a group chosen from;

  - -OR' and -SR' groups, where R' represents a saturated or unsaturated and linear or branched $C_1$ to $C_{20}$ hydrocarbon group, or a saturated cyclic $C_3$ to $C_{10}$ hydrocarbon group, or an aryl or heteroaryl group; and
  - -NR'R" groups, where R' is as defined above, while R" represents either a hydrogen atom, or a saturated or unsaturated and linear or branched $C_1$ to $C_{20}$ hydrocarbon group, or a saturated cyclic $C_3$ to $C_{10}$ hydrocarbon group, or still an aryl or heteroaryl group;

- $R^2$ represents a hydrogen atom or a group identical to or different from $R^1$, chosen from:

  - -OR' and -SR' groups, where R' represents a saturated or unsaturated and linear or branched $C_1$ to $C_{20}$ hydrocarbon group, or a saturated cyclic $C_3$ to $C_{10}$ hydrocarbon group, or an aryl or heteroaryl group; and
  - -NR'R" groups, where R' is as defined above, while R" represents either a hydrogen atom, or a saturated or unsaturated and linear or branched $C_1$ to $C_{20}$ hydrocarbon group, or a saturated cyclic $C_3$ to $C_{10}$ hydrocarbon group, or still an aryl or heteroaryl group; while

- $R^3$ represents:

  - an acyl group, or
  - a saturated or unsaturated and linear or branched $C_1$ to $C_{20}$ hydrocarbon group, or
  - a saturated cyclic $C_3$ to $C_{10}$ hydrocarbon group, or

- a saturated $C_3$ to $C_{10}$ heterocyclic group, or still
- an aryl or heteroaryl group;

or one of its salts, in solution in a solvent.

**12.** Liquid scintillator according to claim 11, in which the 1,8-naphthalimide derivative exhibits an emission wavelength of between 350 and 550 nm and better still between 380 and 470 nm.

**13.** Liquid scintillator according to claim 11 or claim 12, in which the 1,8-naphthalimide derivative corresponds to the general formula (I) in which $R^1$ represents an -OR' or -SR' group, where R' represents a saturated or unsaturated and linear or branched $C_1$ to $C_{20}$ hydrocarbon group, or a saturated cyclic $C_3$ to $C_{10}$ hydrocarbon group, or still an aryl or heteroaryl group, while $R^2$ represents a hydrogen atom.

**14.** Liquid scintillator according to claim 13, in which the 1,8-naphthalimide derivative corresponds to the general formula (I) in which $R^1$ represents a $C_1$ to $C_{20}$ and better still $C_1$ to $C_{10}$ alkoxy, alkenyloxy or alkylsulphanyl group.

**15.** Liquid scintillator according to any one of claims 11 to 14, in which the 1,8-naphthalimide derivative corresponds to the general formula (I) in which $R^3$ represents an aryl or heteroaryl group substituted in the 2 and 5 positions, with respect to the nitrogen atom of the naphthalimide ring system, by a branched $C_3$ to $C_6$ alkyl group.

**16.** Liquid scintillator according to claim 15, in which the 1,8-naphthalimide derivative corresponds to the general formula (I) in which $R^3$ represents a di(t-butyl)phenyl group.

**17.** Liquid scintillator according to any one of claims 11 to 16, in which the 1,8-naphthalimide derivative corresponds to any one of the specific formulae below:

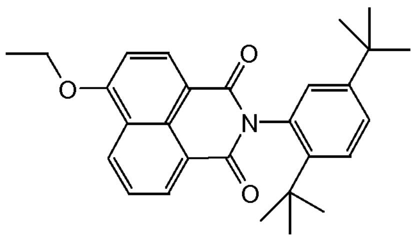

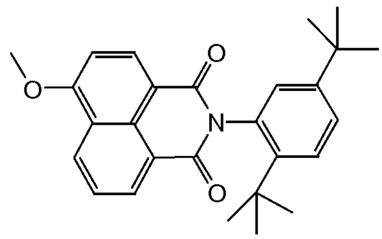

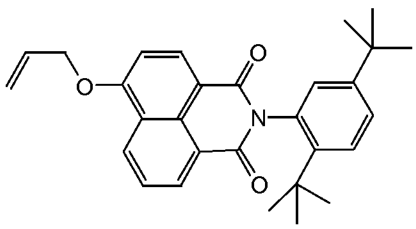

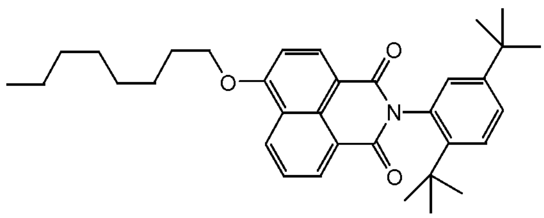

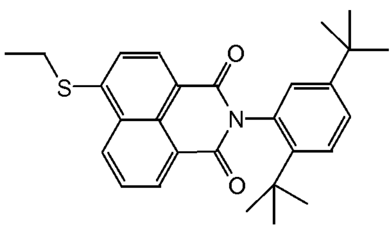

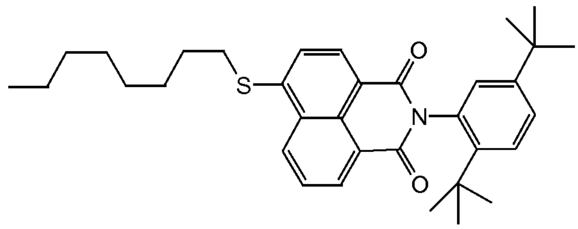

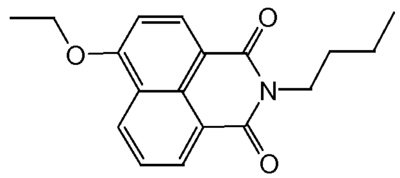

**18.** Liquid scintillator according to any one of claims 11 to 17, in which the 1,8-naphthalimide derivative is present at a concentration at least equal to 3 g/l and preferably of between 8 and 12 g/l.

**19.** 1,8-Naphthalimide derivative which corresponds to the general formula (I) below:

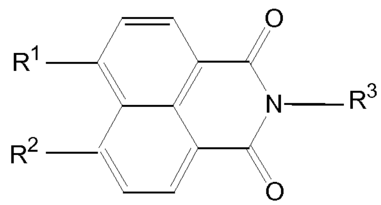

(I)

in which:

- $R^1$ represents a group chosen from -OR' and -SR' groups, where R' represents a saturated or unsaturated and linear or branched $C_1$ to $C_{20}$ hydrocarbon group, or a saturated cyclic $C_3$ to $C_{10}$ hydrocarbon group, or still an aryl or heteroaryl group;
- $R^2$ represents a hydrogen atom or a group also chosen from -OR' and -SR' groups, where R' represents a saturated or unsaturated and linear or branched $C_1$ to $C_{20}$ hydrocarbon group, or a saturated cyclic $C_3$ to $C_{10}$ hydrocarbon group, or still an aryl or heteroaryl group; while
- $R^3$ represents an aryl or heteroaryl group substituted in the 2 and 5 positions, with respect to the nitrogen atom of the naphthalimide ring system, by a branched $C_3$ to $C_6$ alkyl group;

and its salts.

**20.** 1,8-Naphthalimide derivative according to claim 19, which corresponds to the general formula (I), in which $R^2$ represents a hydrogen atom.

**21.** 1,8-Naphthalimide derivative according to claim 19 or claim 20, which corresponds to the general formula (I) in which $R^1$ represents a $C_1$ to $C_{20}$ and better still $C_1$ to $C_{10}$ alkoxy, alkenyloxy or alkylsulphanyl group.

**22.** 1,8-Naphthalimide derivative according to claim 19, which corresponds to the general formula (I) in which $R^3$ represents a di(t-butyl)phenyl group.

**23.** 1,8-Naphthalimide derivative according to any one of claims 19 to 22, which corresponds to any one of the specific formulae below:

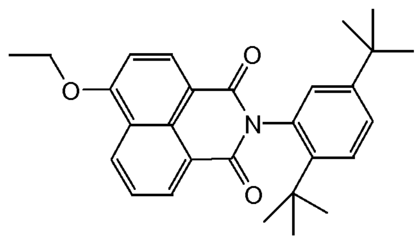

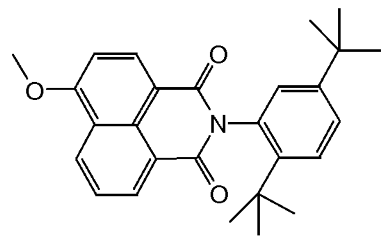

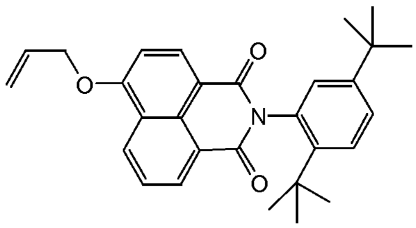

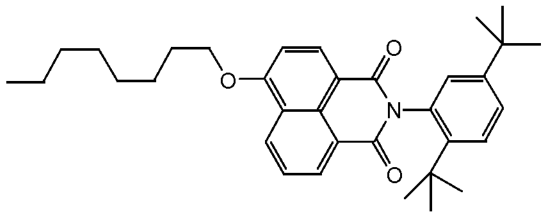

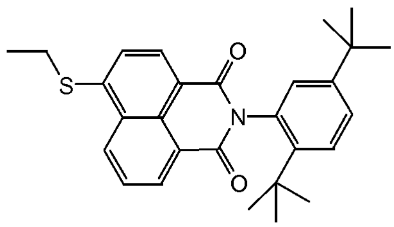

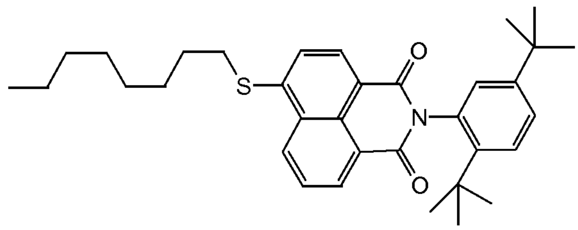

**24.** 1,8-Naphthalimide derivative according to claim 23, which corresponds to the specific formula below:

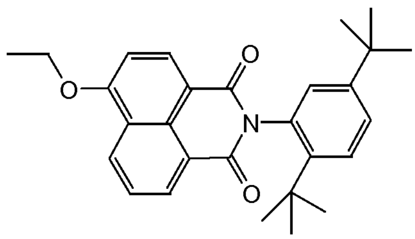

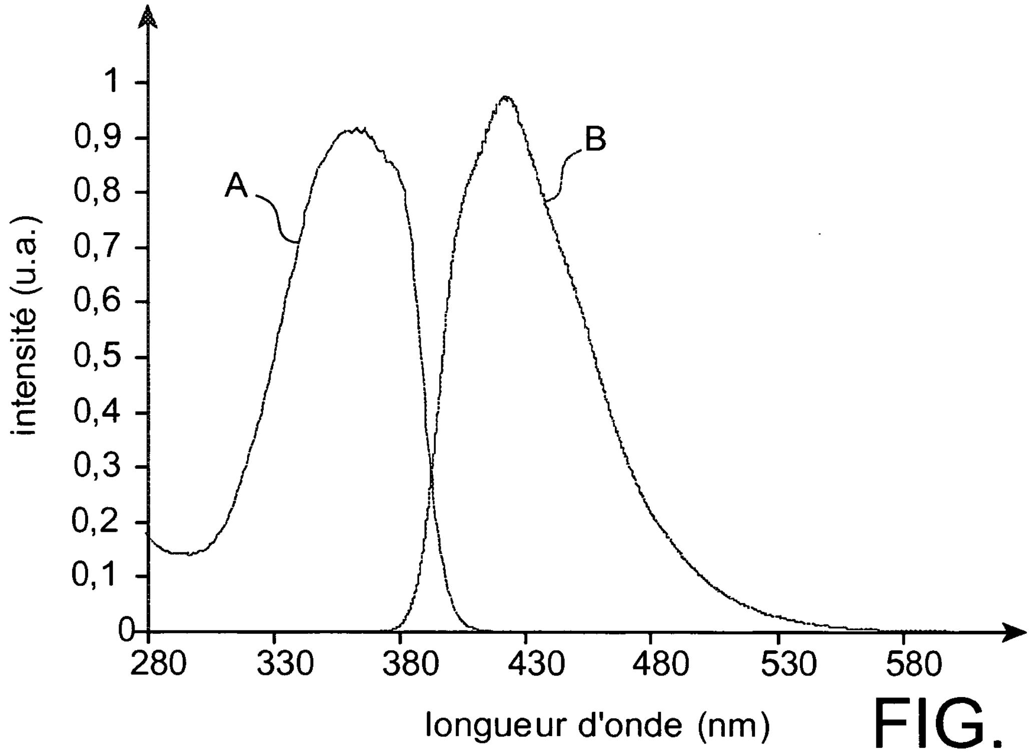
1
0,9
0,8
0,7
0,6
0,5
0,4
0,3
0,2
0,1
0
intensité (u.a.)
A
B
280
330
380
430
480
530
580
longueur d'onde (nm)

FIG. 1

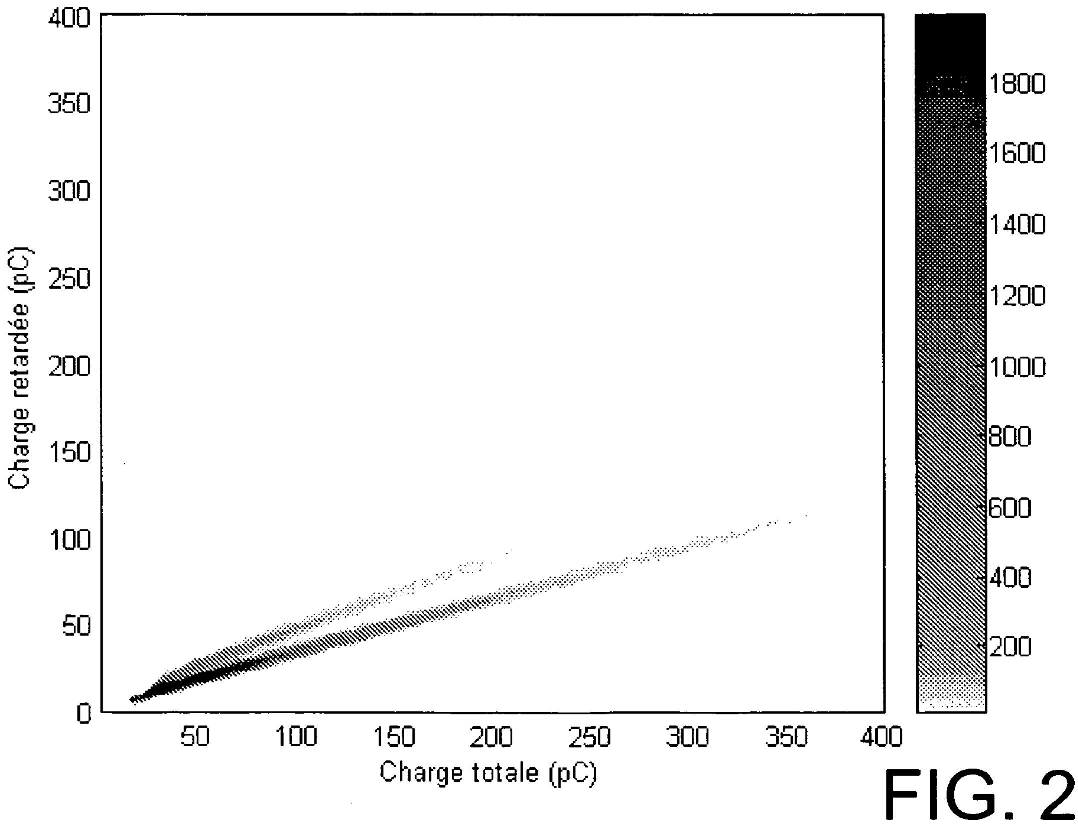
400
350
300
250
200
150
100
50
0
Charge retardée (pC)
50
100
150
200
250
300
350
400
Charge totale (pC)
1800
1600
1400
1200
1000
800
600
400
200

FIG. 2

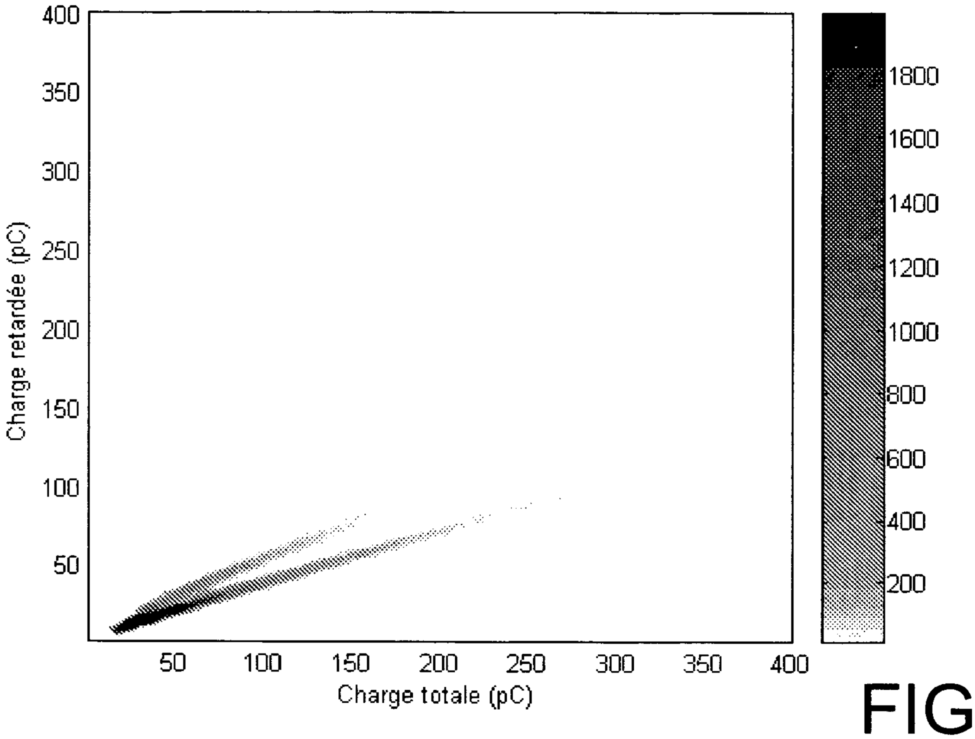
400
350
300
250
200
150
100
50
Charge retardée (pC)
50 100 150 200 250 300 350 400
Charge totale (pC)
1800
1600
1400
1200
1000
800
600
400
200

FIG. 3

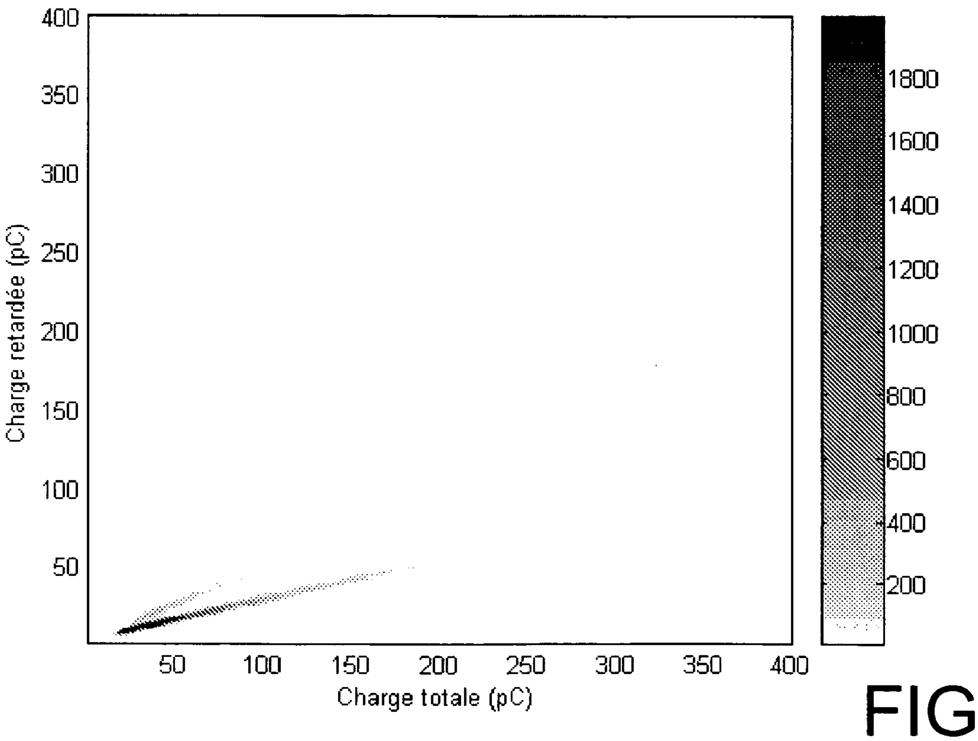
400
350
300
250
200
150
100
50
Charge retardée (pC)
50 100 150 200 250 300 350 400
Charge totale (pC)
1800
1600
1400
1200
1000
800
600
400
200

FIG. 4

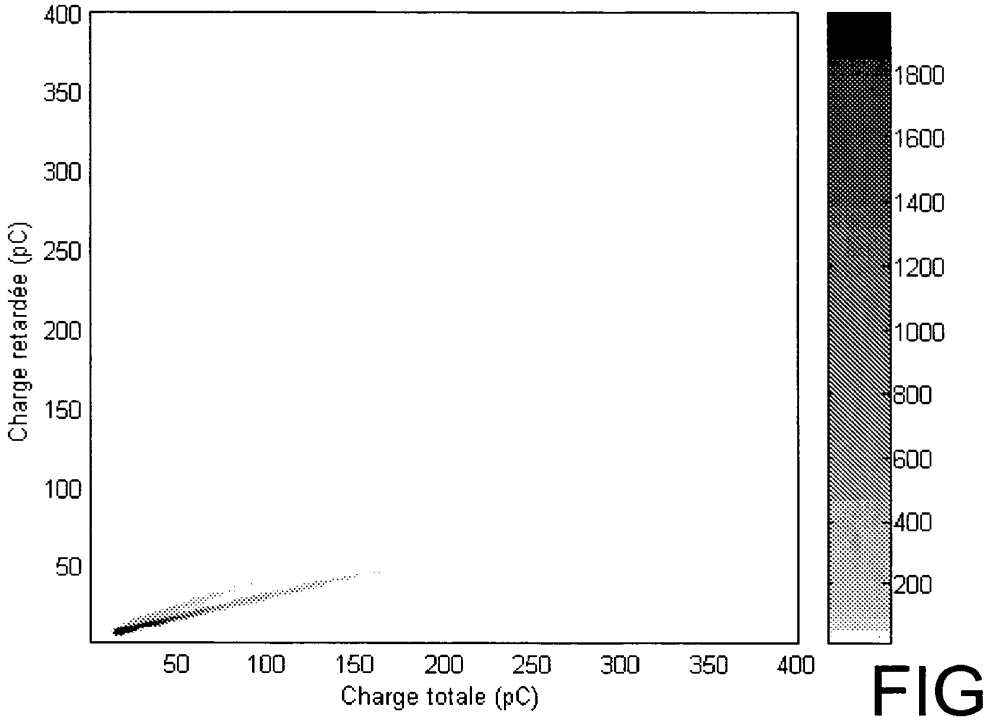
400
350
300
250
200
150
100
50
Charge retardée (pC)
50
100
150
200
250
300
350
400
Charge totale (pC)
1800
1600
1400
1200
1000
800
600
400
200

FIG. 5

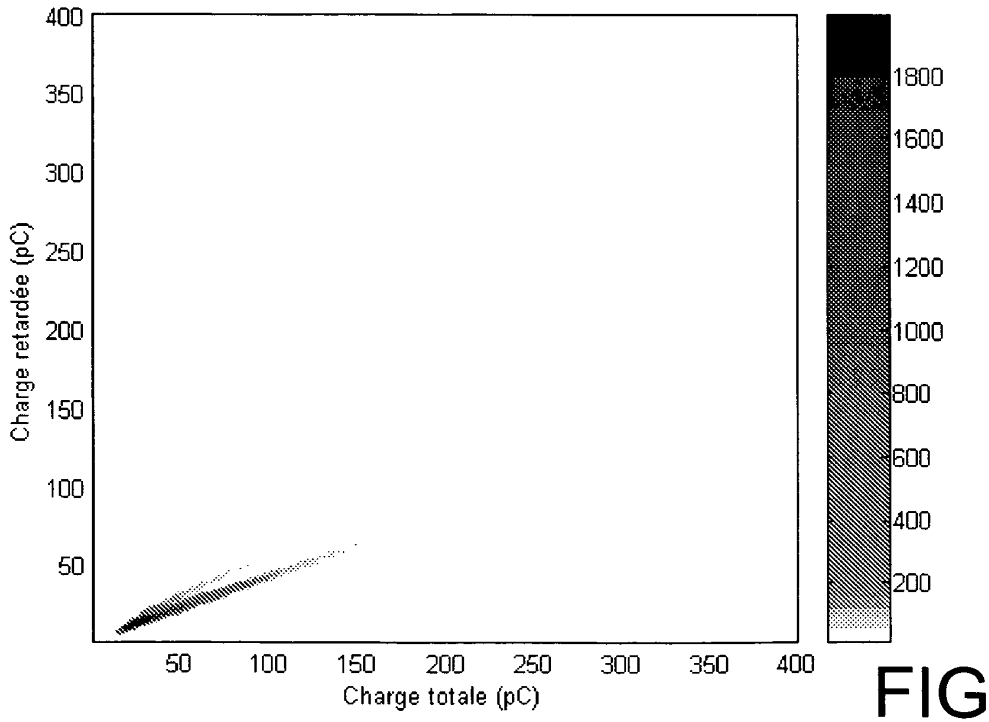
400
350
300
250
200
150
100
50
Charge retardée (pC)
50
100
150
200
250
300
350
400
Charge totale (pC)
1800
1600
1400
1200
1000
800
600
400
200

FIG. 6

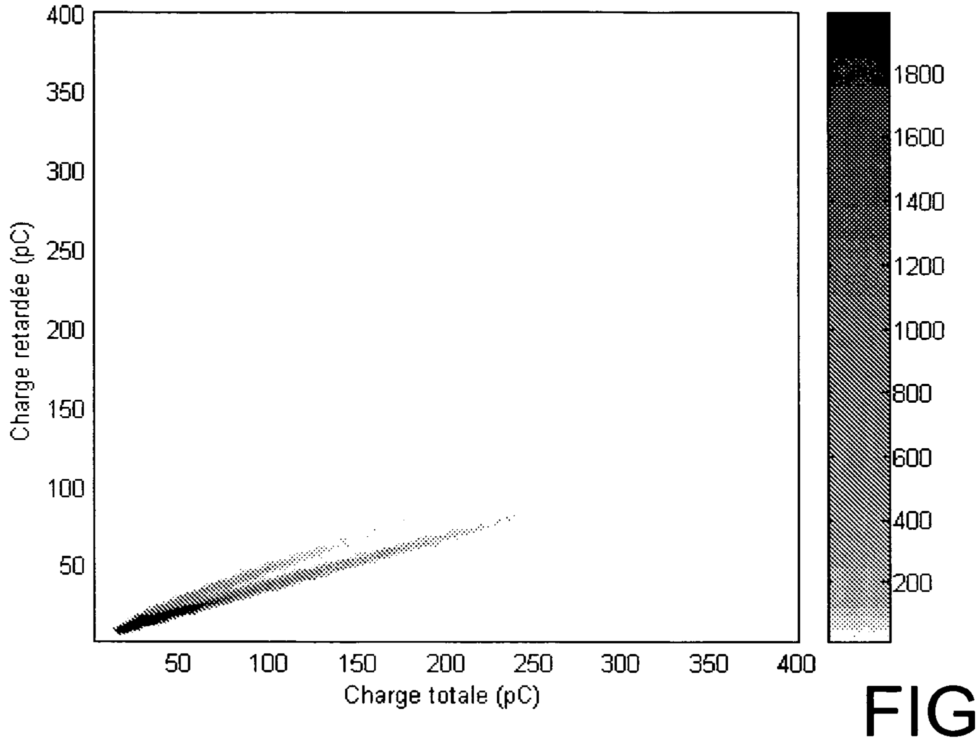
400
350
300
250
200
150
100
50
Charge retardée (pC)
50 100 150 200 250 300 350 400
Charge totale (pC)
1800
1600
1400
1200
1000
800
600
400
200

FIG. 7

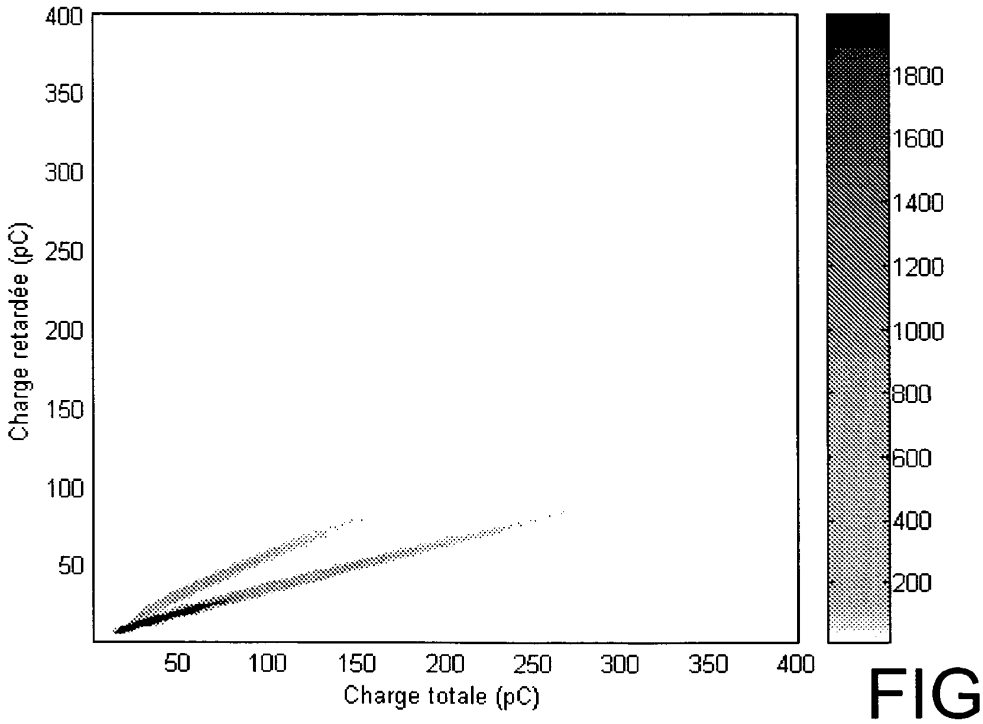
400
350
300
250
200
150
100
50
Charge retardée (pC)
50 100 150 200 250 300 350 400
Charge totale (pC)
1800
1600
1400
1200
1000
800
600
400
200

FIG. 8

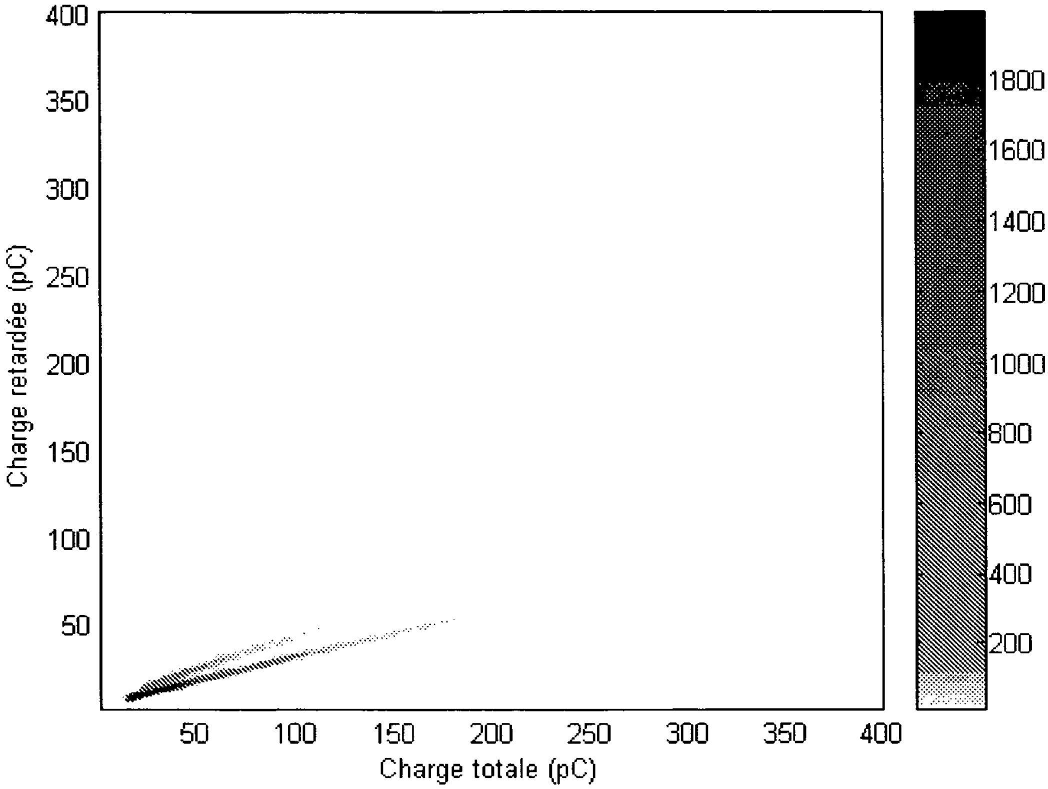
400
350
300
250
200
150
100
50
Charge retardée (pC)
50
100
150
200
250
300
350
400
Charge totale (pC)
1800
1600
1400
1200
1000
800
600
400
200

FIG. 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **B. KESANLI et al.** *Appl. Phys. Lett.,* 2006, vol. 89, 214104
- **M. I. BARNIK et al.** *Nucl. Instrum. Methods Phys. Res., Sect. A,* 2000, vol. 449, 537-545
- **S. NORMAND et al.** *Nucl. Instrum. Methods Phys. Res. A,* 2002, vol. 484, 342-350
- **M. MOSZYŃSKI et al.** *Nucl. Instrum. Methods Phys. Res. A,* 1994, vol. 350, 226-234